# EUROPEAN PATENT SPECIFICATION

(11) **EP 2 742 950 B1**
(45) Date of publication and mention of the grant of the patent: **08.03.2017**
(21) Application number: 11870806.4
(22) Date of filing: 22.12.2011
(51) Int. Cl.: A61K 38/39, A61K 38/17, C07K 14/78, A61P 35/00

(54) **PHARMACEUTICAL COMPOSITION CONTAINING FIBULIN-3 PROTEIN AS AN ACTIVE INGREDIENT FOR INHIBITING THE GROWTH OF CANCER STEM CELLS**
PHARMAZEUTISCHE ZUSAMMENSETZUNG MIT FIBULIN-3 PROTEIN ALS WIRKSTOFF ZUR HEMMUNG DES WACHSTUMS VON KREBSSTAMMZELLEN
COMPOSITION PHARMACEUTIQUE CONTENANT UNE PROTÉINE FIBULINE-3 COMME PRINCIPE ACTIF POUR L'INHIBITION DE LA CROISSANCE DE CELLULES SOUCHES CANCÉREUSES

(30) Priority: 09.08.2011 KR 20110079033
(43) Date of publication of application: 18.06.2014
(73) Proprietor: Korea Atomic Energy Research Institute, Daejeon 305-353 (KR)
(72) Inventor: KIM, In-Gyu, Daejeon 305-755 (KR); SHIN, Byungchul, Seoul 135-280 (KR); KIM, Kug Chan, Daejeon 305-761 (KR); KIM, Seo Yeon, Daejeon 306-130 (KR); KIM, Min Jung, Daejeon 302-282 (KR); LEE, So Yong, Cheongju-si Chungcheongbuk-do 361-812 (KR)
(74) Representative: Grünecker Patent- und Rechtsanwälte PartG mbB
(86) International application number: PCT/KR2011/010001
(87) International publication number: WO 2013/022157

(56) References cited:
- US-A1- 2006 094 054
- US-A1- 2007 220 621
- US-B2- 7 892 760
- EN-LIN SONG ET AL: "EFEMP1 expression promotes angiogenesis and accelerates the growth of cervical cancer", GYNECOLOGIC ONCOLOGY, vol. 121, no. 1, 15 December 2010 (2010-12-15), pages 174-180, XP028164676, ISSN: 0090-8258, DOI: 10.1016/J.YGYNO.2010.11.004 [retrieved on 2010-11-16]
- SADR-NABAVI, A. ET AL.: 'Decreased expression of angiogenesis antagonist EFEMP1 in sporadic breast cancer is caused by aberrant promoter methylation and points to an impact of EFEMP 1 as molecular biomarker' INT. J. CANCER. vol. 124, no. 7, 01 April 2009, pages 1727 - 1735, XP055138127
- YUE, W. ET AL.: 'Frequent inactivation ofRAMP2, EFEMP1 and Dutt1 in lung cancer by promoter hypermethylation' CLIN. CANCER RES. vol. 13, no. 15, 01 August 2007, pages 4336 - 4344, XP055138128
- ALBIG, A. R. ET AL.: 'Fibulins 3 and 5 antagonize tumor angiogenesis in vivo' CANCER RES. vol. 66, no. 5, 01 March 2006, pages 2621 - 2629, XP055138131

## Description

### BACKGROUND OF THE INVENTION

### 1. Field of the Invention

The present invention relates to a pharmaceutical composition for inhibiting the growth of cancer stem cells comprising fibulin-3 protein produced by the method of the invention or a method for inhibiting the growth of cancer stem cells using the same.

### 2. Description of the Related Art

Up to date, it has been reported that cancer is developed when a normal cell is mutated by a certain reason given from surrounding environment. Thus, the development of anti-cancer agents or the treatment of cancer has been targeting a certain gene that is specifically or excessively expressed in none other than cancer cells. However, this attempt has not been very successful to eliminate cancer cells in those patients having metastatic cancer or recurrent cancer. In the course of studying those patients, it has been found out that there is a certain cell found in cancer cells that causes cancer and this cell displays characteristics of normal stem cell. This cell has been named "cancer stem cell" because it shows characteristics of stem cell. Paradigm of the cancer stem cell is attributed to a specific cell group showing pulprint potency and self renewal [B.M. Boman, M.S. Wicha, Cancer stem cells: a step toward the cure, J. Clin. Oncol. 26(2008) 2795-2799]. The cancer stem cell was first identified in acute myeloid leukemia, and ever since it has been identified in general solid tumors including breast cancer, etc. [D. Bonnet, J.E. Dick, Human acute myeloid leukemia is organized as hierarchy that originates from a primitive hematopoietic cell. Nat. Med 3(1997)730- 737; M. Al-Hajj, M.F. Clarke, Self-renewal and tumor stem cells. Oncogene 23(2003)7274-7284]. Such cancer stem cell has resistance against anti-cancer agents developed so far. 80 - 90% of cancer cells do not have self renewal capacity, but 10 - 20% of cancer cells (cancer stem cells) are not killed and survived the anti-cancer agents.

Therefore, to avoid the recurrence and metastasis of cancer and to eliminate cancer completely, the limitation of recent cancer treatment methods which targeting cancer cells only has to be overcome and the novel anti-cancer agent being able to kill cancer stem cells by targeting those cancer stem cells having characteristics of stem cell has to be developed.

Today's cancer treatment methods are divided largely into surgical operation, chemo-therapy, and radio-therapy. Except surgical operation, chemo-therapy and radio-therapy are performed with a variety of medicines and different radiations whose applications are though limited in the endurance of human body. Even though such chemo-therapy and radio-therapy demonstrated excellent treating effect in animal test, the limited application of chemo-therapy and radio-therapy in human, considering the endurance of human body, cut down the clinical effect, suggesting the limitation not only in applicable subjects but also in effect. The appearance of cancer cells having resistance against anti-cancer agents or cytotoxicity of each therapy can also be a reason of such limitation. Cisplatin is the most efficient anti-cancer agent, so far, among 30 kinds of anti-cancer drugs clinically used, which is known to be effective in treating testis cancer, ovarian cancer, lung cancer, head and neck cancer, bladder cancer, stomach cancer, uterine cervical cancer, etc. (Teni Boulikas, Oncology Reports,10:1663-1682, 2003). However, according to recent reports, cancers showing resistance against cisplatin have been confirmed. Such cancer cells showing resistance against anti-cancer drugs have been a problem and particularly worry us of high risk of cancer recurrence after the treatment. Thus, combined treatment of cisplatin and other chemicals or combined treatment of cisplatin and other methods to regulate intracellular protein expression has been actively attempted (Tito Fojo, Oncogene, 22: 7512-7523, 2003). Radio-therapy is one of anti-cancer treatment methods which is to treat various human cancers by irradiating a required amount of radiation to affected area. The radio-therapy, however, decreases human hematopoietic function and suppresses immune system with lowering the efficiency in cancer treatment. When cancer cells acquire resistance owing to the repeated irradiation, such radio-therapy is limited in use for cancer treatment.

Markers for cancer stem cells are CD133 (prominin-1 or AC133) and CD44 (hyaluronate receptor or P-glycoprotein 1), etc. Aldehyde dehydrogenases 1 (ALDH1) is the recent addition to those markers. ALDH is a detoxifying enzyme that oxidizes intracellular aldehyde, which demonstrates a great resistance against alkylating agent or oxidative stress [Blood 87(1996) 1097-1103; Chem. Biol. Interact. 143-144(2003)5-22]. ALDH converts retinol (vitamin A) into retinoic acid that is the most active form of retinoids playing an important role in treating and preventing cancer, suggesting that ALDH is also involved in cell growth and proliferation. It has been known that ALDH contains 19 genes, which are classified into ALDH1, 2, and 3 classes. Among them, only ALDH1 has retinal dehydrogenase activity that converts retinal into retinoic acid. Along with ALDH1A1, ALDH1A3 (retinaldehyde dehydrogenase 3) has highly efficient activity [Biochem. Biophys. Acta 1790(2009) 1660-1664]. So, the activity of ALDH1 is efficiently utilized to isolate subpopulation of cancer stem cells from lung cancer cell line, etc. [Mol. Cancer. Res. 7(2009) 330-338].

Wnt/β-catenin pathway, which has been recently reported to play an important role in the generation of cancer stem cell (Nature Reviews Cancer 5, 744-749 (September 2005)) that is one of causes of cancer recurrence, is evolutionally well conserved pathway. This pathway regulates cell growth, morphology, and motility during embryogenesis. This pathway also plays an important role in maintaining homeostasis of tissues including intestines and brain by regulating somatic stem cells and the surroundings thereof. It has also been reported that wnt is activated in cancer cells and the activation inhibits the activity of glycogen synthase kinase 3 (GSK3), leading to the accumulation of β-catenin, the substrate of GSK3. Such abnormal wnt pathway induces neoplastic proliferation.

Fibulin proteins are the proteins conjugated with elastic extracellular matrix (ECM) to contribute to intercellular interaction or cell-matrix interaction, which are classified into 6 types from type 1 to type 6. Some Fibulin proteins have been proposed as a new gene target to inhibit tumor. Recently, fibulin-5 and corresponding fibulin-3 have been identified to be involved in cell growth regulation and some kinds of cancers. According to the recent report, fibulin-5 inhibits tumor, and is involved in cell growth, cell invasion, vascular endothelial growth factor and tumor angiogenesis [J. Biol. Chem. 277(2002): 27367-27377; DNA. Cell Biol. 23(2004): 367-379; Future Oncology 1(2005):23-35; Cancer Res. 69(2009): 6339-6346]. In the meantime, fibulin-3 is involved in cell growth regulation, and tumor angiogenesis, and hence inhibits some kinds of tumors [Cancer Res. 66(2006) 2621-2629]. However, the effect of fibulin-3 on cancer stem cells which induce tumor development or help tumor to maintain its characteristics to resist anti-cancer treatment has not been disclosed, yet.
US2006094054 discloses that fibulin-3 enhances tumorigenicity in fibrosarcoma cells. US2007220621 discloses that fibulin-3 promotes angiogenesis in HeLa cells in a mouse model.

The present inventors isolated cancer stem cells from H460 and A549 cells, which are non-small cell lung cancer cells having different sensitivity to cytotoxicity such as permeability and ionizing radiation, by using ALDH1 activity as a marker and then confirmed that there was difference in fibulin-3 expression between in ALDH1 active cells and ALDH1 inactive cells. When fibulin-3 expression was increased in the cells separated above, β-catenin giving the characteristics of cancer stem cells and infiltration related factors MMP-2 and MMP-7 were decreased. That is, the growth of cancer stem cells was inhibited by fibuline-3. The medium in which fibulin-3 was over-expressed gave the same result. In addition, the inventors constructed fibulin-3 purification vector to purify fibulin-3 protein. The inventors further confirmed that the purified fibulin-3 protein inhibited the growth of the non-small-cell lung cancer cell line A549, the breast cancer cell line MDA-MB231, and the cancer stem cell line active ALDH1. The present inventors completed this invention by confirming that the said fibulin-3 could be effectively used as a composition for inhibiting the growth of cancer stem cells.

### SUMMARY OF THE INVENTION

It is an object of the present invention to provide fibulin-3 protein having the activity of inhibiting cancer stem cell growth of lung or breast cancer.

It is further an object of the present invention to provide a pharmaceutical composition for inhibiting lung or breast cancer stem cell growth comprising fibulin-3 protein or fibulin-3 over-expressing cell line constructed by transforming fibulin-3 gene or the culture medium of the same as an active ingredient.

It is also an object of the present invention to provide means for inhibiting lung or breast cancer stem cell growth containing the step of treating cancer stem cells with an effective dose of fibulin-3 protein or fibulin-3 over-expressing cell line constructed by transforming fibulin-3 gene or the culture medium of the same.

It is also an object of the present invention to provide means for inhibiting lung or breast cancer stem cell growth containing the step of administering a pharmaceutically effective dose of fibulin-3 protein to a subject having cancer stem cells.

It is also an object of the present invention to provide means for inhibiting lung or breast cancer stem cell growth containing the step of administering a pharmaceutically effective dose of fibulin-3 over-expressing cell line constructed by transforming fibulin-3 gene or the culture medium of the same to a subject having cancer stem cells.

It is also an object of the present invention to provide a use of fibulin-3 protein for the preparation of a pharmaceutical composition for inhibiting cancer stem cell growth of lung or breast cancer.

In addition, it is also an object of the present invention to provide a use of fibulin-3 over-expressing cell line constructed by transforming fibulin-3 gene or the culture medium of the same for the preparation of a pharmaceutical composition for inhibiting cancer stem cell growth of lung or breast cancer.

To achieve the above objects, the present invention provides fibulin-3 protein having the activity of inhibiting cancer stem cell growth.

The present disclosure also teaches a preparation method of fibulin-3 protein having the activity of inhibiting cancer stem cell growth comprising the following steps:
1) constructing the vector encoding fibulin-3 protein;
2) transfecting cells with the vector of step 1); and
3) purifying fibulin-3 protein from the cell culture medium of step 2).

The present invention further provides a pharmaceutical composition for inhibiting lung or breast cancer stem cell growth comprising fibulin-3 protein having the activity of inhibiting cancer stem cell growth as an active ingredient.

The present invention also provides means for inhibiting lung or breast cancer stem cell growth containing the step of treating cancer stem cells with an effective dose of fibulin-3 protein.

The present invention also provides a pharmaceutical composition for inhibiting lung or breast cancer stem cell growth comprising fibulin-3 over-expressing cell line constructed by transforming fibulin-3 gene or the culture medium of the same as an active ingredient.

The present invention also provides means for inhibiting lung or breast cancer stem cell growth containing the step of treating cancer stem cells with fibulin-3 over-expressing cell line constructed by transforming fibuoin-3 gene or the culture medium of the same.

The present invention also provides means for inhibiting lung or breast cancer stem cell growth containing the step of administering a pharmaceutically effective dose of fibulin-3 protein to a subject having cancer stem cells.

The present invention also provides means for inhibiting lung or breast cancer stem cell growth containing the step of administering a pharmaceutically effective dose of fibulin-3 over-expressing cell line constructed by transforming fibulin-3 gene or the culture medium of the same to a subject having cancer stem cells.

The present invention also provides a use of fibulin-3 protein for the preparation of a pharmaceutical composition for inhibiting cancer stem cell growth of lung or breast cancer.

In addition, the present invention provides a use of fibulin-3 over-expressing cell line constructed by transforming fibulin-3 gene or the culture medium of the same for the preparation of a pharmaceutical composition for inhibiting cancer stem cell growth of lung or breast cancer.

### ADVANTAGEOUS EFFECT

As explained hereinbefore, the fibulin-3 protein of the present invention can inhibit cancer stem cell growth by suppressing β-catenin and invasion related factors (MMP-2 and MMP-7) giving the characteristics of cancer stem cell in cancer stem cells separated from non-small-cell lung cancer cells by using ALDH1 activity. In addition, the fibulin-3 protein of the present invention can inhibit the growth of non-small-cell lung cancer cells, breast cancer cells, and cancer stem cells. Therefore, the protein can be effectively used for the preparation of a pharmaceutical composition for inhibiting cancer stem cell growth.

### BRIEF DESCRIPTION OF THE DRAWINGS

The application of the preferred embodiments of the present invention is best understood with reference to the accompanying drawings, wherein:
Figure 1 is a diagram illustrating the vector for protein purification constructed by conjugating histidine protein nucleotide sequence to c-terminal of fibulin-3 gene.
Figure 2 is a diagram illustrating the separation of ALDH1 active cells and ALDH1 inactive cells from A549 cells by using FACS after staining A549 cells with Aldefluor;
   AL(+): ALDH1 active cells; and,
   AL(-): ALDH1 inactive cells.
Figure 3 is a diagram illustrating the over-expression of histidine-conjugated figulin-3 gene in CHO-K1 cells, confirmed by RT-PCR and Western blotting;
   RT-PCR: Reverse Transcription Polymerase Chain Reaction;
   Western: Western blotting;
   pcDNA: control CHO-K1 cells in which empty vector was over-expressed; and,
   His-fibulin3: CHO-K1 cells in which histidine-conjugated fibulin-3 was over-expressed.
Figure 4 is a diagram illustrating A549 cells, ALDH1 active cells separated from A549 cells, and ALDH1 inactive cells separated from A549 cells, confirmed by colony formation analysis;
   A549: colony formation of A549 cells;
   ALDH+: colony formation of ALDH1 active cells; and,
   ALDH-: colony formation of ALDH1 inactive cells.
Figure 5 illustrates the expressions of ALDH1A1, β-catenin and fibulin-3 in ALDH1 active cells and in ALDH1 inactive cells, each separated from A549 cells, confirmed by RT-PCR. At this time, the expressions in A549 cells were used as controls;
   Alde(+): ALDH1 active cells;
   Alde(-) : ALDH1 inactive cells; and,
   ALDH1A1: ALDH1.
Figure 6 illustrates the expression of β-catenin in A549 cells and H460 cells, confirmed by Western blotting and RT-PCR;
   RT-PCR: result of RT-PCR; and,
   Western: result of Western blotting.
Figure 7 illustrates the expressions of β-catenin and fibulin-3 in A549 cells, fibulin-3 over-expressing A549 cells, H460 cells, and fibulin-3 suppressed H460 cells;
   A549: A549 cells;
   A549/V.C: control A549 cells in which control vector was expressed;
   A549/pcFBLN3: A549 cells in which fibulin-3 over-expressing vector was expressed;
   H460: H460 cells;
   H460/siCtl: control H460 cells in which si-control vector was expressed;
   H460/siFBLN3: H460 cells in which fibulin-3 suppressing vector was expressed;
   FBLN 3: fibulin-3;
   RT-PCR: result of RT-PCR; and,
   Western: result of Western blotting.

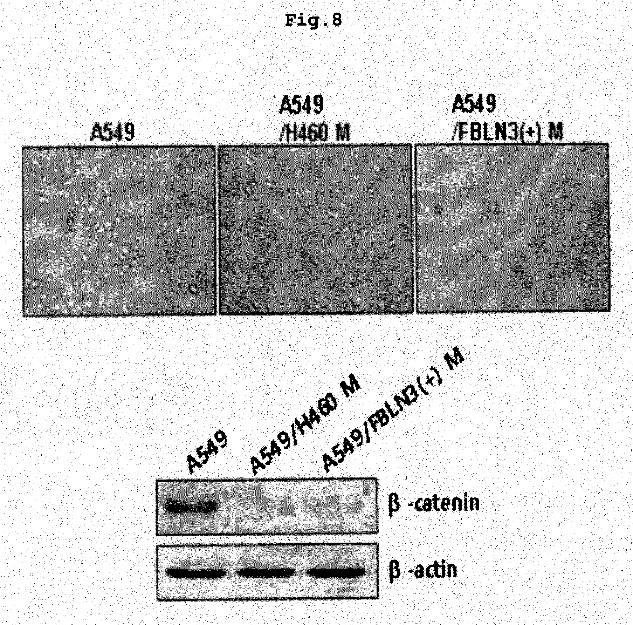

Figure 8 is a photomicrograph illustrating the A549 cells treated with fibulin-3 over-expressing A549 cell culture medium or fibluin-3 over-expressing H460 cell culture medium and a diagram illustrating the expression of β-catenin confirmed by Western blotting;
   A549: A549 cells;
   A549/H460 M: A549 cells treated with H460 culture medium; and,
   A549/FBLN3(+) M: A549 cells treated with fibulin-3 over-expressing A549 cell culture medium.

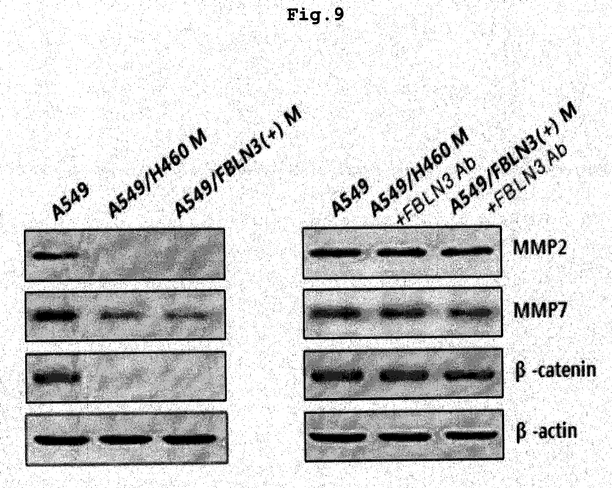

Figure 9 illustrates the expressions of β-catenin, MMP-2, and MMP-7 in A549 cells treated with fibulin-3 over-expressing A549 cell culture medium or fibluin-3 over-expressing H460 cell culture medium, or co-treated with fibulin-3 over-expressing A549 cell
   A549/H460 M: A549 cells treated with H460 culture medium; and,
   A549/FBLN3(+) M: A549 cells treated with fibulin-3 over-expressing A549 cell culture medium.
Figure 9 illustrates the expressions of β-catenin, MMP-2, and MMP-7 in A549 cells treated with fibulin-3 over-expressing A549 cell culture medium or fibluin-3 over-expressing H460 cell culture medium, or co-treated with fibulin-3 over-expressing A549 cell culture medium or fibluin-3 over-expressing H460 cell culture medium along with fibulin-3 antibody, confirmed by Western blotting;
   A549: A549 cells;
   A549/H460 M: A549 cells treated with H460 culture medium;
   A549/FBLN3(+) M: A549 cells treated with fibulin-3 over-expressing A549 cell culture medium;
   A549/H460 M +FBLN3 Ab: A549 cells treated with H460 culture medium and fibulin-3 antibody; and,
   A549/FBLN3(+) M +FBLN3 Ab: A549 cells treated with fibulin-3 over-expressing A549 cell culture medium and fibulin-3 antibody.

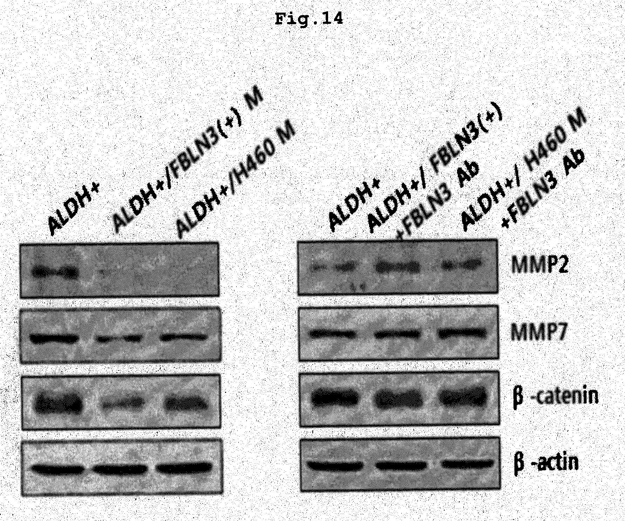

Figure 14 illustrates the expressions of β-catenin, MMP-2, and MMP-7 in ALDH1 active cells treated with fibulin-3 over-expressing A549 cell culture medium or fibluin-3 over-expressing H460 cell culture medium, or co-treated with fibulin-3 over-expressing A549 cell culture medium or fibluin-3 over-expressing H460 cell culture medium along with fibulin-3 antibody, confirmed by Western blotting;
   ALDH+: ALDH1 active cells;
   ALDH+/FBLN3(+) M: ALDH1 active cells treated with fibulin-3 over-expressing A549 cell culture medium;
   ALDH+/H460 M: ALDH1 active cells treated with H460 culture medium;
   ALDH+/FBLN3(+) M +FBLN3 Ab: ALDH1 active cells treated with fibulin-3 over-expressing A549 cell culture medium and fibulin-3 antibody; and,
   ALDH+/H460 M +FBLN3 Ab: ALDH1 active cells treated with H460 culture medium and fibulin-3 antibody.

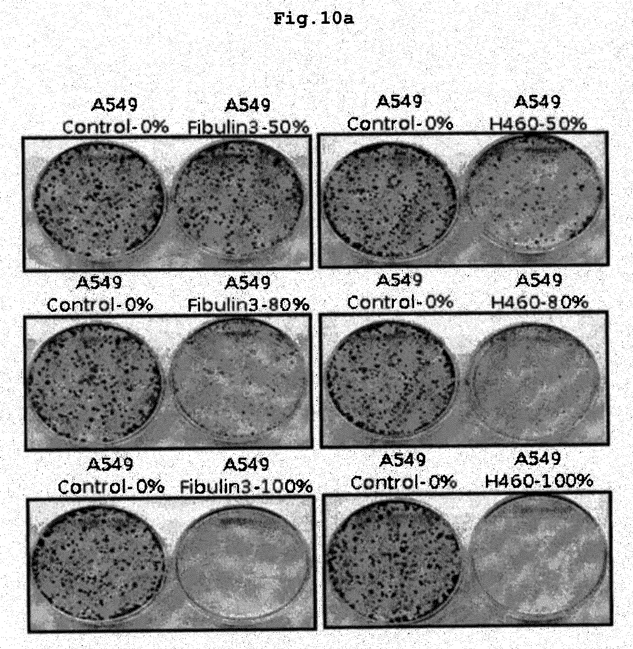

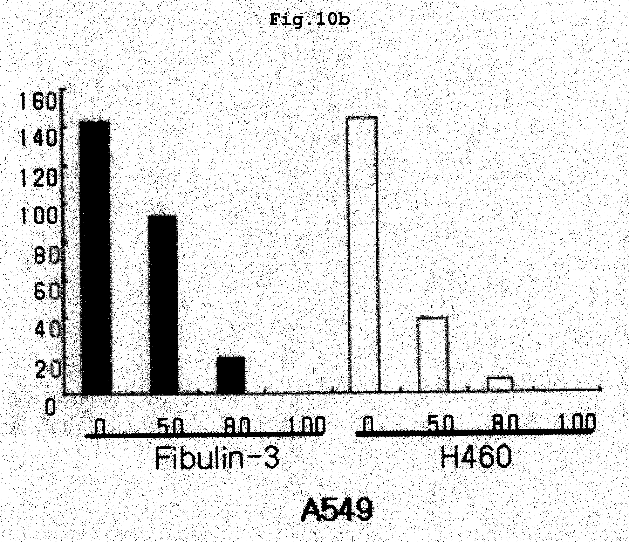

Figure 10 illustrates the colony formation of A549 cells confirmed after treating the cells with different concentrations of fibulin-3 over-expressing A549 cell culture medium or fibluin-3 over-expressing H460 cell culture medium;
   a: photograph of colony;
   b: graph displaying the number of colonies;
   Control: control treated with A549 cell culture medium;
   fibulin3-%: ratio of fibulin-3 over-expressing medium in the mixed medium of fibulin-3 over-expressing A549 cell culture medium and A549 growth medium; and,
   H460-%: ratio of H460 cell culture medium in the mixed medium of H460 cell culture medium and A549 growth medium.

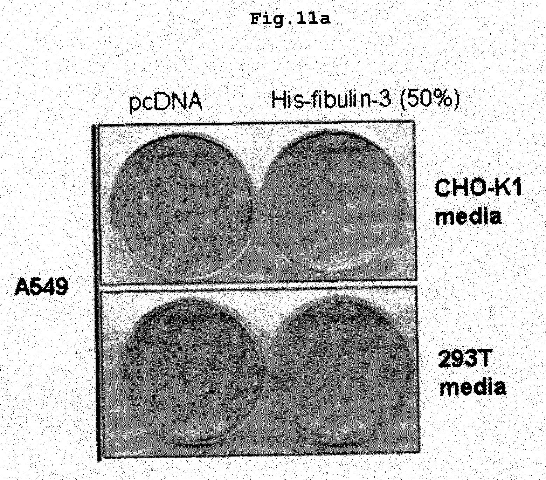

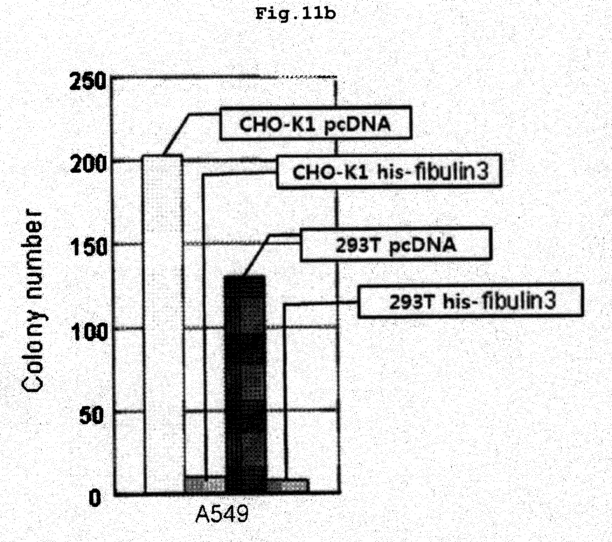

Figure 11 illustrates the colony formation of A549 cells confirmed after treating the cells with his-fibulin-3 vector over-expressing CHO-K1 cell culture medium or his-fibulin-3 vector over-expressing 293T cell culture medium by 50%;
   a: photograph of colony;
   CHO-K1 medium: his-fibulin-3 vector over-expressing CHO-K1 cell culture medium;
   293T medium: his-fibulin-3 vector over-expressing 293T cell culture medium; and,
   b: graph displaying the number of colonies.

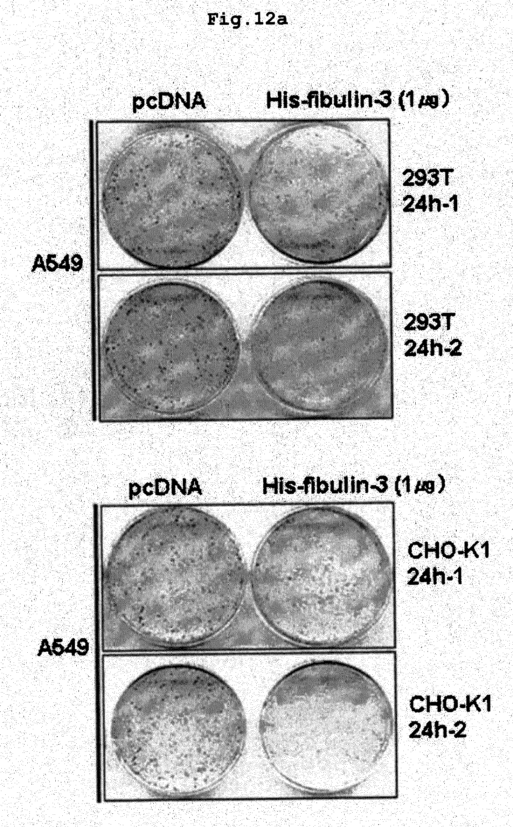

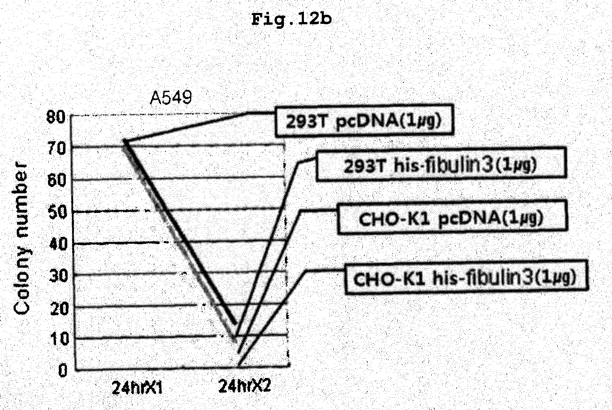

Figure 12 illustrates the colony formation of A549 cells confirmed after treating the cells with the purified fibulin-3 protein;
   a: photograph of colony;
   CHO-K1 24h-1: A549 cells treated with 1 ug of fibulin protein purified from CHO-K1 cells over-expressing his-fibulin-3 vector 24 hours after the purification;
   CHO-K1 24h-2: A549 cells treated twice with 1 ug of fibulin protein purified from CHO-K1 cells over-expressing his-fibulin 3 vector at 24 hour intervals;
   293T 24h-1: A549 cells treated with 1 ug of fibulin protein purified from 293T cells over-expressing his-fibulin-3 vector 24 hours after the purification;
   293T 24h-2: A549 cells treated twice with 1 ug of fibulin protein purified from 293T cells over-expressing his-fibulin 3 vector at 24 hour intervals;
   pc-DNA: control; and,
   b: graph displaying the number of colonies.

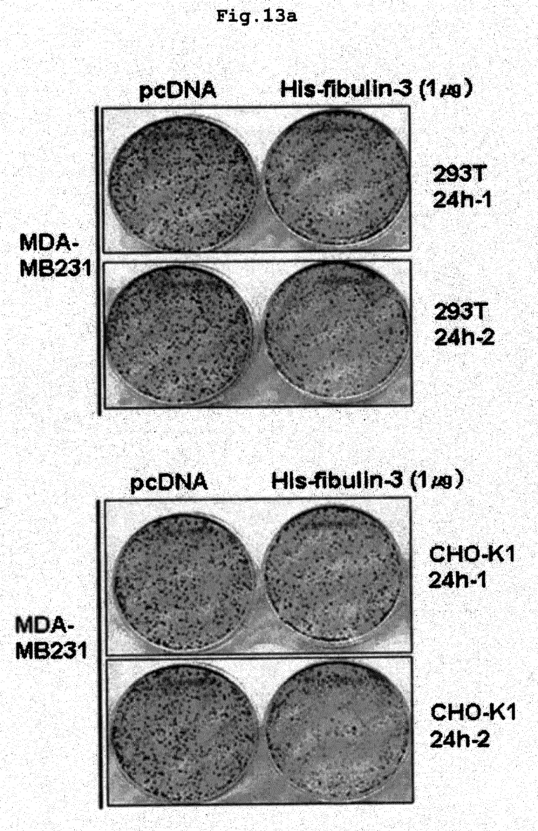

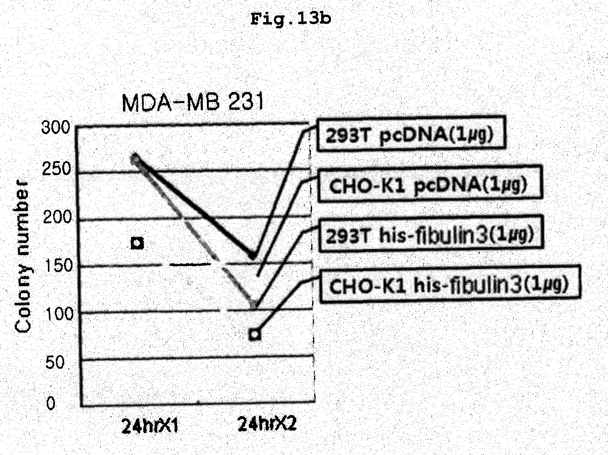

Figure 13 illustrates the colony formation of MDA-MB231 cells confirmed after treating the cells with the purified fibulin-3 protein;
   a: photograph of colony;
   CHO-K1 24h-1: MDA-MB231 cells treated with 1 ug of fibulin protein purified from CHO-K1 cells over-expressing his-fibulin-3 vector 24 hours after the purification;
   CHO-K1 24h-2: MDA-MB231 cells treated twice with 1 ug of fibulin protein purified from CHO-K1 cells
   293T 24h-2: MDA-MB231 cells treated twice with 1 ug of fibulin protein purified from 293T cells over-expressing his-fibulin 3 vector at 24 hour intervals;
   pc-DNA: control; and,
   b: graph displaying the number of colonies.

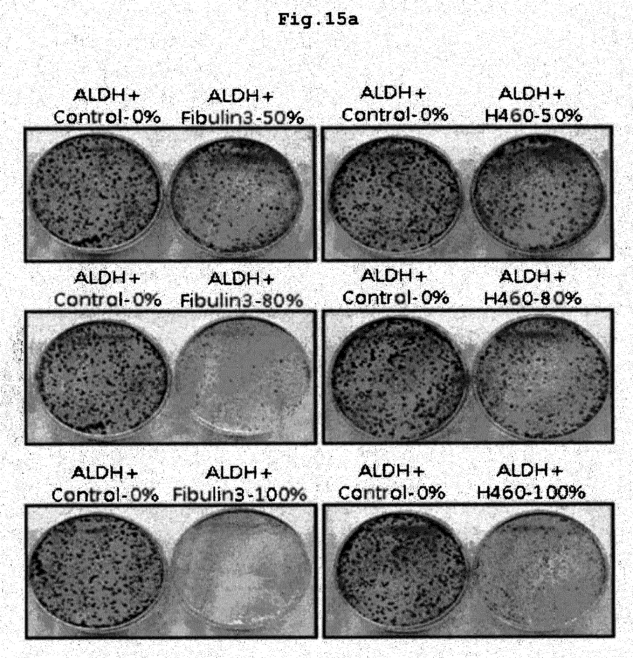

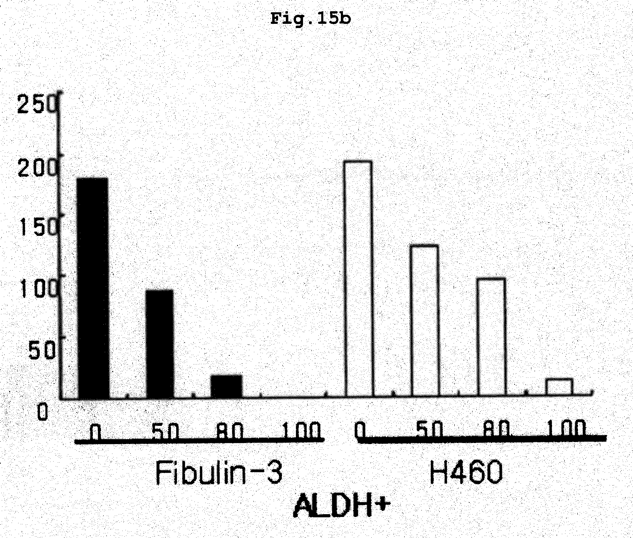

Figure 15 illustrates the colony formation of ALDH1 active cells confirmed after treating the cells with fibulin-3 over-expressing A549 cell culture medium and fibulin-3 over-expressing H460 cell culture medium at different concentrations;
   a: photograph of colony;
   b: graph displaying the number of colonies;
   ALDH1+: ALDH1 active cells;
   control: control group treated with ALDH1 cell growth medium;
   fibulin3-%: ratio of fibulin-3 over-expressing medium in the mixed medium of fibulin-3 over-expressing A549 cell culture medium and A549 cell growth medium; and,
   H460-%: ratio of H460 cell culture medium in the mixed medium of H460 cell culture medium and ALDH1 growth medium.

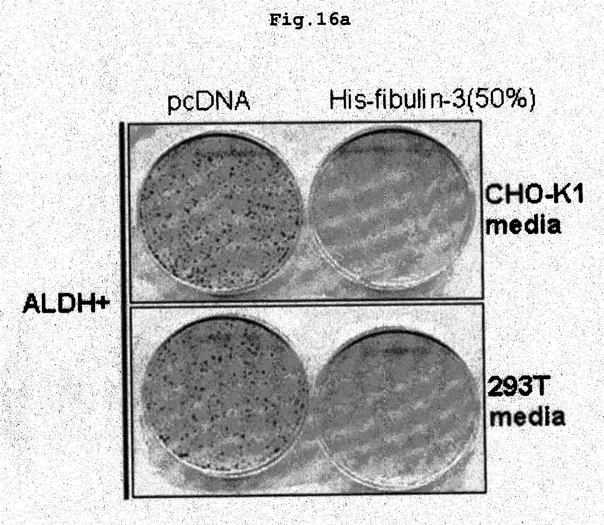

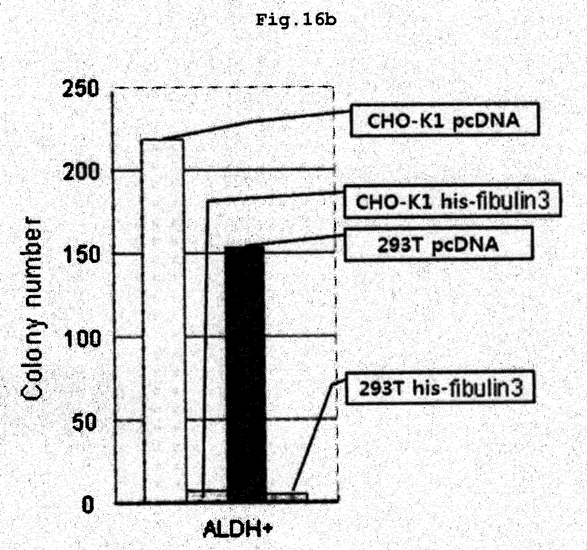

Figure 16 illustrates the colony formation of ALDH1 active cells confirmed after treating the cells with his-fibulin-3 vector over-expressing CHO-K1 cell culture medium or his-fibulin-3 vector over-expressing 293T cell culture medium by 50%;
   a: photograph of colony;
   ALDH+: ALDH1 active cells;
   CHO-K1 medium: his-fibulin-3 vector over-expressing CHO-K1 cell culture medium;
   293T medium: his-fibulin-3 vector over-expressing 293T cell culture medium; and,
   b: graph displaying the number of colonies.

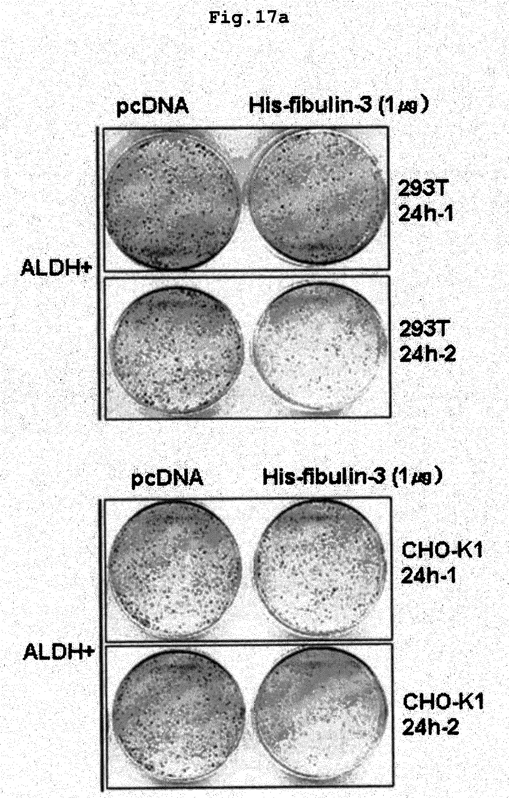

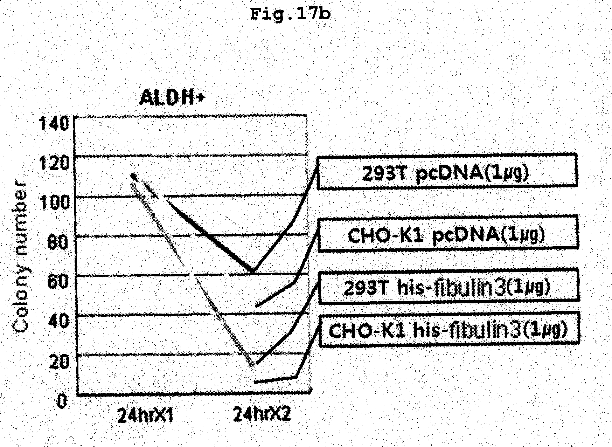

Figure 17 illustrates the colony formation of ALDH1 active cells confirmed after treating the cells with the purified fibulin-3 protein;
   a: photograph of colony;
   ALDH+: ALDH1 active cells;
   CHO-K1 24h-1: ALDH1 active cells treated with 1 ug of fibulin protein purified from ALDH1 active cells over-expressing his-fibulin-3 vector 24 hours after the purification;
   CHO-K1 24h-2: ALDH1 active cells treated twice with 1 ug of fibulin protein purified from ALDH1 active cells over-expressing his-fibulin-3 vector at 24 hour intervals;
   293T 24h-1: ALDH1 active cells treated with 1 ug of fibulin protein purified from 293T cells over-expressing his-fibulin-3 vector 24 hours after the purification;
   293T 24h-2: ALDH1 active cells treated twice with 1 ug of fibulin protein purified from 293T cells over-expressing his-fibulin 3 vector at 24 hour intervals;
   pc-DNA: control; and,
   b: graph displaying the number of colonies.

### DESCRIPTION OF THE PREFERRED EMBODIMENTS

Hereinafter, the present invention is described in detail.

The present invention provides fibulin-3 protein having the activity of inhibiting cancer stem cell growth of lung or breast cancer.

The said fibulin-3 protein preferably contains one of the amino acid sequences selected from the group consisting of:
1) amino acid sequence represented by SEQ. ID. NO: 1;
2) amino acid sequence comprising
   the sequence represented by SEQ. ID. NO: 1

The said cancer herein is lung cancer or breast cancer, and the lung cancer herein is preferably non-small-cell lung cancer.

The present disclosure also provides a preparation method of fibulin-3 protein having the activity of inhibiting cancer stem cell growth comprising the following steps:
1) constructing the vector encoding fibulin-3 protein;
2) transfecting cells with the vector of step 1); and
3) purifying fibulin-3 protein from the cell culture medium of step 2).

The said cancer herein is lung cancer or breast cancer, and the lung cancer herein is preferably non-small-cell lung cancer.

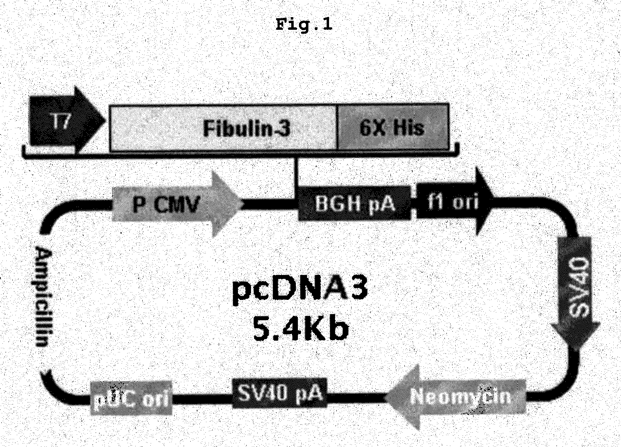

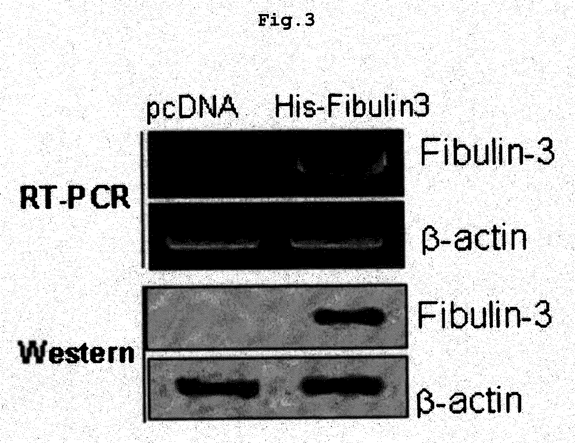

In a preferred embodiment of the present invention, the present inventors constructed fibulin-3 vector by conjugating histidine protein to c-terminal for the purification of protein (see Figure 1). To confirm the expression of the said vector, the vector was introduced respectively into CHO-K1 cells (Korea Research Institute of Bioscience and Biotechnology, Daejeon, Korea) and 293T cells (Hanyang University, Seoul, Korea), followed by PCR and Western blotting to examine the over-expression of fibulin-3 protein (see Figure 3). The transformed cell culture medium was also treated to A549 cells (non-small-cell lung cancer cells) and ALDH1 active cells (cancer stem cells). As a result, the growth of A549 cells and ALDH1 active cells was all inhibited after the treatment of the medium (see Figures 11 ∼ 16). In addition, fibulin-3 protein was purified by using the transformed cell line, followed by investigation of cancer cell growth inhibiting effect of the purified fibulin-3 protein. As a result, it was confirmed that fibulin-3 had the activity of inhibiting the growth of A549 cells (non-small-cell lung cancer cells), ALDH1 active cells (cancer stem cells), and MDA-MB231 (breast cancer cells) (see Figure 12, Figure 17, and Figure 13).

Therefore, fibulin-3 protein can be effectively used for the inhibition of the growth of cancer cells and cancer stem cells.

The present invention also provides a pharmaceutical composition for inhibiting cancer stem cell growth comprising the fibulin-3 protein of the invention as an active ingredient.

The said fibulin-3 protein preferably contains one of the amino acid sequences selected from the group consisting of:
1) amino acid sequence represented by SEQ. ID. NO: 1;
2) amino acid sequence comprising
   the sequence represented by SEQ. ID. NO: 1

The said cancer herein is lung cancer or breast cancer, and the lung cancer herein is preferably non-small-cell lung cancer.

The cancer stem cell is preferably selected by using the marker selected from the group consisting of such cancer stem cell markers as CD133 (prominin-1; AC133), CD44 (hyaluronate receptor; P-glycoprotein 1) and ALDH1, and is more preferably selected by using ALDH1, but not always limited thereto.

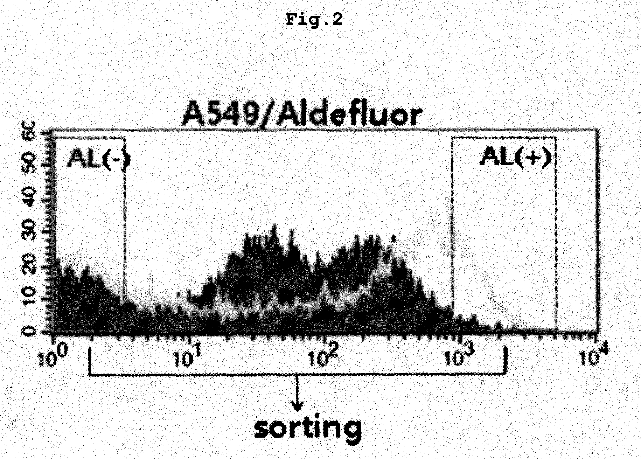

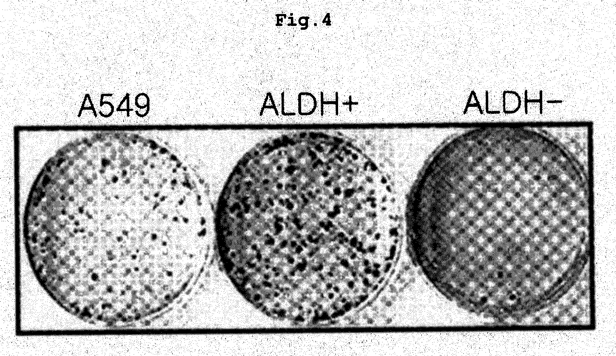

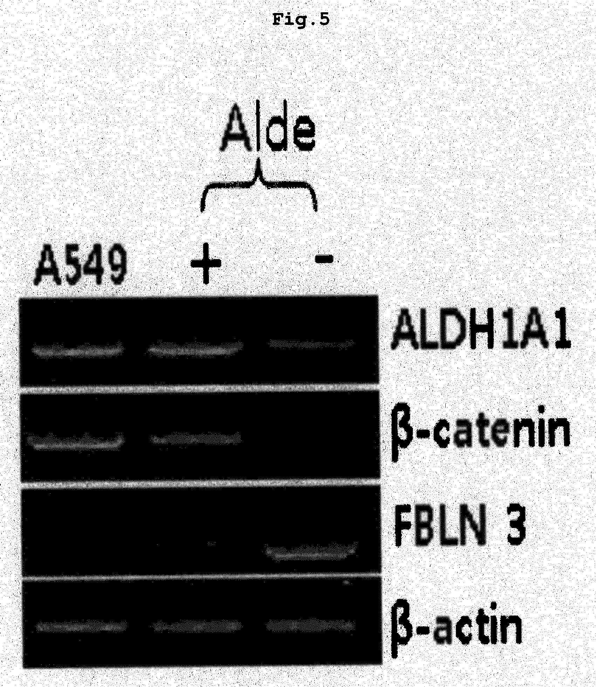

In a preferred embodiment of the present invention, the present inventors distinguished and selected cancer stem cells from A549 cells (non-small-cell lung cancer cells) by using ALDEFLUOR product (stem cell) based on ALDH1 activity (see Figure 2). The present inventors also investigated the growth of ALDH1 active cells and the growth of ALDH1 inactive cells isolated by colony formation test in order to confirm the difference between non-small-cell lung cancer cells and cancer stem cells. As a result, the growth of ALDH1 active cells was more peculiar than the growth of A549 cells, while the growth of ALDH1 inactive cells was weaker than that of the control A549 cells, indicating that cancer stem cells having ALDH1 activity were the cause of the growth of non-small-cell lung cancer (see Figure 4). In addition, the present inventors performed RT-PCR and Western blotting in order to confirm the expressions of ALDH1, fibulin-3, and β-catenin in ALDH1 active cells and in ALDH1 inactive cells as well. As a result, unlike in ALDH1 inactive cells, the expression of β-catenin, which is an important factor for the development and growth of tumor, was increased in ALDH1 active cells. However, the expression of fibulin-3 therein was reduced (see Figure 5). That is, since β-catenin known to play an important role in the growth of cancer stem cells, the major cause of cancer growth, was up-regulated while fibulin-3 was down-regulated, fibulin-3 was confirmed to be effectively used for the suppression of cancer stem cells.

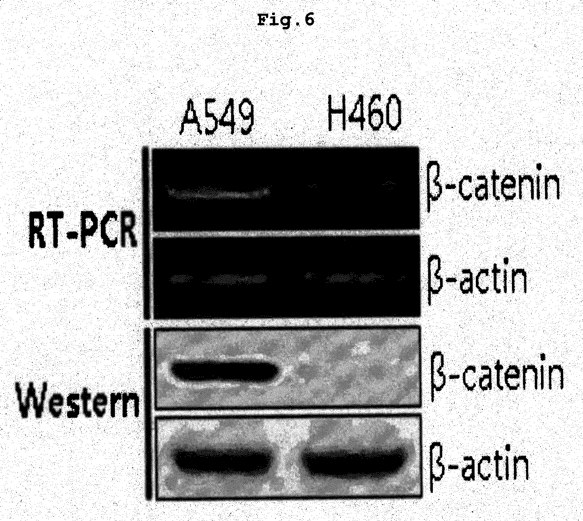

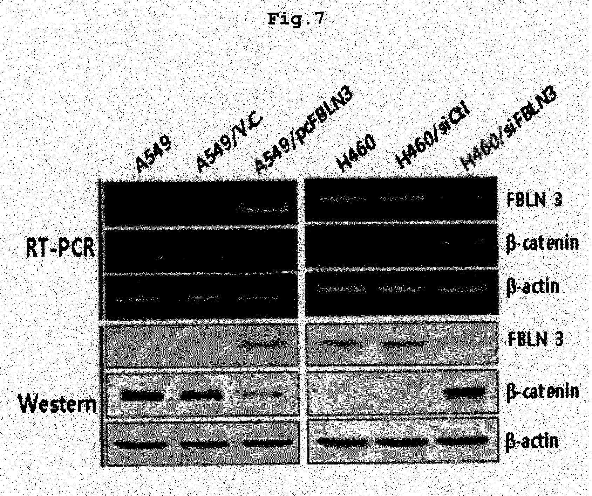

In another preferred embodiment of the present invention, the present inventors introduced fibuin-3 over-expressing vector into A549 cells demonstrating higher expression of β-catenin, in order to confirm the expression of β-catenin induced by fibulin-3. In the meantime, the inventors inhibited the expression of fibulin-3 in H460 cells showing relatively lower expression of β-catenin with fibulin-3 suppressing siRNA. As a result, the expression of β-catenin in A549 cells over-expressing fibulin-3 was reduced, while the expression of β-catenin in H460 cells in which fibulin-3 was suppressed was increased (see Figures 6 and 7). Therefore, it was confirmed that the over-expressed fibulin-3 inhibited β-catenin playing an important role in the growth of cancer stem cells, and hence fibulin-3 could be confirmed to be an effective candidate for inhibiting cancer stem cell growth.

In another preferred embodiment of the present invention, the present inventors treated A549 cells, the non-small cell lung cancer cells, with fibulin-3 over-expressing A549 cell culture medium and fibulin-3 over-expressing H460 cell culture medium in order to investigate the inhibitory effect of fibulin-3 on the expressions of β-catenin, MMP-2, and MMP-7, important factors for the development and growth of tumor. As a result, it was confirmed that apoptosis was induced in the cells treated with the said medium, observed under microscope, and the expressions of β-catenin, MMP-2, and MMP-7 were all reduced, compared with those of the control (see Figures 8 and 9). To re-confirm that the above result was attributed to fibulin-3, the experiment using fibulin-3 antibody was also performed. As a result, it was re-assured that fibulin-3 directly inhibited the expressions of β-catenin, MMP-2, and MMP-7. In another preferred embodiment of the present invention, the present inventors investigated the expressions of β-catenin, MMP-2, and MMP-7 induced by fibulin-3 in cancer stem cells. Particularly, ALDH1 active cells were treated with fibulin-3 over-expressing ALDH1 active cell culture medium and fibulin-3 over-expressing H460 cell culture medium, followed by Western blotting. as a result, the expressions of β-catenin, MMP-2, and MMP-7 were reduced in the group treated with the above two media. It was re-assured that the above result was attributed to fibulin-3 by performing the additional experiment using fibulin-3 antibody (see Figure 14). In conclusion, fibulin-3 inhibited directly the expressions of β-catenin, MMP-2, and MMP-7 in cancer stem cells.

The present inventors also performed colony formation test in order to confirm whether the growth of non-small-cell lung cancer cells could be inhibited by fibulin-3. As a result, the growth of A549 cells treated with fibulin-3 over-expressing A549 cell culture medium or the mixed medium comprising fibulin-3 over-expressing H460 cell culture medium was inhibited dose-dependently (see Figure 10). The present inventors performed colony formation test in order to confirm the inhibition of cancer stem cell growth by fibulin-3. As a result, the growth of ALDH1 active cells treated with fibulin-3 over-expressing A549 cell culture medium or the mixed medium comprising fibulin-3 over-expressing H460 cell culture medium was inhibited dose-dependently (see Figure 10). The present inventors induced over-expression of his-fibulin-3 vector designed for the expression of fibulin-3 protein in CHO-K1 cells and 293T cells, which are both easy cells for protein purification, and then cultured thereof for 48 hours. The culture medium was treated to A549 cells, the non-small-cell lung cancer cells, and ALDH1 active cells, the cancer stem cells. As a result, the growth of the cells treated with the medium was inhibited, compared with that of the control cells (see Figure 11 and Figure 16). In addition, to investigate whether or not the growth of cancer cells and cancer stem cells could be suppressed by the purified fibulin-3 protein, A549 cells (non-small-cell lung cancer cells), MDA-MB231 cells (breast cancer cells), and ALDH1 active cells (cancer stem cells) were treated with the purified fibulin-3 protein obtained from CHO-K1 cells or 293T cells transfected with his-fibulin-3 vector designed for the expression of fibulin-3 protein, and then colony formation test was performed. As a result, it was confirmed that the purified fibulin-3 protein inhibited the growth of A549 cells (non-small-cell lung cancer cells), MDA-MB231 cells (breast cancer cells), and ALDH1 active cells (cancer stem cells) (see Figure 12, Figure 13, and Figure 17).

Therefore, since fibulin-3 protein can inhibit cancer stem cell growth factors and invasion factors, fibulin-3 protein can be effectively used as an active ingredient of a pharmaceutical composition for inhibiting cancer stem cell growth.

The pharmaceutically effective dosage of the composition of the present invention can be determined by considering various factors such as administration method, target area, patient condition, etc. Thus, the dosage for human body has to be determined with the consideration of safety and efficiency at the same time. It is also possible to predict the effective dosage based on the effective dosage confirmed by animal test. Various factors that have to be considered for the determination of the effective dosage are described in the following articles: Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The composition of the present invention can include any generally used carrier, diluent, excipient, or a combination of at least two of those. The pharmaceutically acceptable carrier can be any carrier that is able to deliver the composition of the present invention in human body without limitation, which is exemplified by the compounds described in Merck Index, 13^{th} ed., Merck & Co. Inc., such as saline, sterilized water, Ringer's solution, buffered saline, dextrose solution, maltodextrin solution, glycerol, ethanol and a mixture comprising one or more of those components. If necessary, a general additive such as antioxidant, buffer, and bacteriostatic agent can be additionally added. The composition of the present invention can be formulated in different forms including aqueous solutions, suspensions and emulsions for injection, pills, capsules, granules or tablets by mixing with diluents, dispersing agents, surfactants, binders and lubricants. The composition can further be prepared in suitable forms according to ingredients by the following method represented in Remington's Pharmaceutical Science (Mack Publishing Company, Easton PA, 18th, 1990).

The composition of the present invention can contain one or more active ingredients having the same or similar functions. The composition of the present invention can include the said protein by 0.0001 ∼ 10 weight%, and preferably 0.001 ∼ 1 weight% by the total weight of the composition.

The composition of the present invention can be administered orally or parenterally (for example, intravenous, hypodermic, peritoneal or local injection). The effective dosage of the composition can be determined according to weight, age, gender, health condition, diet, administration frequency, administration method, excretion and severity of a disease. The dosage is 0.0001 ∼ 10 mg/Mℓ per day and preferably 0.0001 ∼ 5 mg/Mℓ per day, and administration frequency is once a day or preferably a few times a day.

The present invention also provides means for inhibiting cancer stem cell growth containing the step of treating cancer stem cells with an effective dose of fibulin-3.

The present invention also provides means for inhibiting cancer stem cell growth containing the step of administering a pharmaceutically effective dose of fibulin-3 protein to a subject having cancer stem cells.

The said cancer herein is lung cancer or breast cancer, and the lung cancer herein is preferably non-small-cell lung cancer.

In a preferred embodiment of the present invention, the present inventors performed colony formation test in order to confirm whether or not fibulin-3 could inhibit the growth of cancer stem cells. Particularly, ALDH1 active cells, the cancer stem cells isolated from A549 cells, were treated with fibulin-3 over-expressing ALDH1 active cell culture medium and fibulin-3 enriched H460 cell culture medium, followed by performing colony formation test. As a result, the growth of ALDH1 active cells treated with the said two media was suppressed dose-dependently. In addition, the his-fibulin-3 vector for the purification of fibulin-3 protein was introduced into CHO-K1 cells or 293T cells, from which fibulin-3 protein was purified. The purified fibulin-3 protein was treated to non-small-cell lung cancer cells A549, breast cancer cells MDA-MB231, and cancer stem cells active ALDH1, followed by performing colony formation test. As a result, it was confirmed that the growth of the cells treated with the purified fibulin-3 protein was suppressed.

Therefore, the fibulin-3 protein of the present invention can be effectively used for the method for inhibiting cancer stem cell growth.

The present invention also provides a pharmaceutical composition for inhibiting cancer stem cell growth comprising fibulin-3 over-expressing cell line constructed by transforming fibulin-3 gene or the culture medium of the same as an active ingredient.

The said cancer herein is lung cancer or breast cancer, and the lung cancer herein is preferably non-small-cell lung cancer.

The present invention also provides means for inhibiting cancer stem cell growth containing the step of treating cancer stem cells with fibulin-3 over-expressing cell line constructed by transforming fibuoin-3 gene or the culture medium of the same.

The present invention also provides means for inhibiting cancer stem cell growth containing the step of administering a pharmaceutically effective dose of fibuline-3 over-expressing cell line constructed by transforming fibulin-3 gene or the culture medium of the same to a subject having cancer stem cells.

The said cancer herein is lung cancer or breast cancer, and the lung cancer herein is preferably non-small-cell lung cancer.

In a preferred embodiment of the present invention, the present inventors investigated whether or not the growth of cancer stem cells was inhibited by fibulin-3 over-expressing cell line constructed by transforming fibulin-3 gene or the culture medium of the same. Particularly, fibulin-3 over-expressing vector was over-expressed in 5×10⁵ ALDH1 active cells and 5×10⁵ H460 cells, followed by culture for 48 hours. ALDH1 active cells were treated with the said ALDH1 cell culture medium and H460 cell culture medium, followed by Western blotting. As a result, it was confirmed that the expressions of β-catenin, MMP-2, and MMP-7 involved in the growth of cancer stem cells were reduced. It was re-assured that the above result was attributed to fibulin-3 by performing the additional experiment using fibulin-3 antibody. From the above results, it was confirmed that fibulin-3 over-expressing cell line prepared by transforming fibulin-3 gene or the culture medium thereof could directly inhibit the expressions of β-catenin, MMP-2, and MMP-7.

The present inventors also performed colony formation test in order to confirm whether or not fibulin-3 could inhibit the growth of cancer stem cells. As a result, in fibulin-3 over-expressing medium or in the mixed media with that, ALDH1 active cell growth was inhibited dose dependently. As a result, the growth of ALDH1 active cells treated with the above two fibulin-3 over-expressing media was suppressed dose-dependently. The present inventors also performed colony formation test in order to confirm whether or not the culture medium obtained by the culture of CHO-K1 cells or 293T cells transfected with his-fibulin-3 vector designed for fibulin-3 protein expression and easy for protein purification could inhibit the growth of lung cancer cells, breast cancer cells, and cancer stem cells. As a result, the above culture medium was confirmed to inhibit the growth of lung cancer cells and breast cancer cells.

Therefore, fibulin-3 over-expressing cell line constructed by transforming fibulin-3 gene or the culture medium of the same could inhibit growth factors and invasion factors of cancer stem cells, suggesting that fibulin-3 over-expressing cell line or the culture medium of the same can be effectively used for the preparation of a pharmaceutical composition for inhibiting cancer stem cell growth.

The present invention also provides a use of fibulin-3 protein for the preparation of a pharmaceutical composition for inhibiting cancer stem cell growth of lung or breast cancer.

In addition, the present invention provides a use of fibulin-3 over-expressing cell line constructed by transforming fibulin-3 gene or the culture medium of the same for the preparation of a pharmaceutical composition for inhibiting cancer stem cell growth.

The said fibulin-3 protein preferably contains the amino acid sequence represented by SEQ. ID. NO: 1, but not always limited thereto.

The said cancer herein is lung cancer or breast cancer.

In a preferred embodiment of the present invention, the present inventors purified fibulin-3 protein and then investigated the inhibitory effect of the purified fibulin-3 protein on cancer cell growth. As a result, it was confirmed that the purified fibulin-3 protein inhibited the growth of non-small-cell lung cancer cells A549, cancer stem cells active ALDH1, and breast cancer cells MDA-MB231, suggesting that fibulin-3 protein could be effectively used for inhibiting the growth of cancer cells and cancer stem cells. The present inventors further confirmed that fibulin-3 over-expressing cell line prepared by transforming fibulin-3 gene or the culture medium of the same could directly inhibit the expressions of β-catenin, MMP-2, and MMP-7 involved in the growth of cancer stem cells. In addition, it was confirmed that fibulin-3 over-expressing medium inhibited the growth of lung cancer cells, breast cancer cells, and cancer stem cells. Thus, fibulin-3 over-expressing cell line constructed by transforming fibulin-3 gene or the culture medium of the same could inhibit the growth of cancer stem cells and invasion factors as well. Therefore, fibulin-3 over-expressing cell line constructed by transforming fibulin-3 gene or the culture medium of the same could inhibit growth factors and invasion factors of cancer stem cells, suggesting that fibulin-3 over-expressing cell line or the culture medium of the same can be effectively used for the preparation of a pharmaceutical composition for inhibiting cancer stem cell growth.

Practical and presently preferred embodiments of the present invention are illustrative as shown in the following Examples, Experimental Examples and Manufacturing Examples.

### Example 1: Construction of fibulin-3 over-expressing vector

### <1-1> Construction of fibulin-3 gene over-expressing clone

The present inventors constructed fibulin-3 gene over-expressing clone in order to induce over-expression of fibulin-3 gene in A549 cells.

Particularly, total RNA was extracted from non-small-cell lung cancer cells H460 (ATCC no. HTB-177) by using High Pure RNA Isolation Kit (Roche, Germany), followed by reverse transcription polymerase chain reaction (RT-PCR) with 1 µg of the total RNA using Transcriptor First Strand cDNA Synthesis Kit (Roche, Germany). To synthesize cDNA, PCR was performed at 55°C for 30 minutes and at 85°C for 5 minutes. Using the prepared cDNA as a template, PCR was performed as follows; predenaturation at 94°C for 5 minutes, denaturation at 94°C for 1 minute, annealing at 56°C for 1 minute, extension at 72°C for 1 minute 30 seconds, 30 cycles from denaturation to extension, and final extension at 72°C for 5 minutes. At this time, the forward primer 5'-ATATAAGCTTATGTTGAAAGCCC-3' (SEQ. ID. NO: 2) and the reverse primer 5'-ATATCTCGAGCTAAAATGAAAATGGCCCC-3' (SEQ. ID. NO: 3) designed to contain *Hind*III recognition site at 5'-end and *Xho*I recognition site at 3'-end were used. As a result, 1,482 bp fibulin-3 gene was obtained. The obtained PCR product and pcDNA3.1 vector (Invitrogen, USA) were digested with restriction enzymes respectively, followed by ligation using ligase to construct fibulin-3 over-expressing pcDNA3.1/fibulin-3 vector.

### <1-2> Construction of his-fibulin-3 vector for fibulin-3 purification

The present inventors constructed fibulin-3 clone attached with histidine protein on its C-terminal for the purification of the protein.

Particularly, PCR was performed using 0.1 µg of the fibulin-3 clone DNA constructed in Example <1-1> as a template with primers designed to express histidine protein next to fibulin-3. PCR was performed as follows; predenaturation at 94°C for 5 minutes, denaturation at 94°C for 1 minute, annealing at 60°C for 1 minute, extension at 72°C for 1 minute 30 seconds, 30 cycles from denaturation to extension, and final extension at 72°C for 5 minutes. At this time, the forward primer 5'-ATATAAGCTTATGTTGAAAGCCC-3' (SEQ. ID. NO: 14) and the reverse primer 5'-CCGGCTCGAGCTAGTGGTGGTGGTGGTGGTGAAATGAAAATGG-3' (SEQ. ID. NO: 15) designed to contain *EcoRI* recognition site at 5'-end and *Xho*I recognition site at 3'-end along with histidine (his) protein nucleotide sequence were used. As a result, fibulin-3 gene conjugated with histidine protein nucleotide sequence was obtained. The obtained PCR product and pcDNA3.1 vector (Invitrogen, USA) were digested with restriction enzymes respectively, followed by ligation using ligase to construct pcDNA3.1/his-fibulin-3 vector for the purification of fibulin-3 protein conjugated with his protein (Figure 1).

### Example 2: Preparation and purification of fibulin-3 over-expressing medium

### <2-1> Preparation of fibulin-3 over-expressing medium

The present inventors prepared the culture medium in which the cells over-expressing fibulin-3 mediated by the fibulin-3 over-expressing vector of Example <1-1> were cultured.

Particularly, 5×10⁵ cells/Mℓ of A549 cells were transfected with 2 µg of the fibulin-3 over-expressing vector prepared in Example <1-1> by using lipofectamine 2000 in penicillin-streptomycin free medium (Hyclone). After 4 ∼ 6 hours of the reaction, the medium was replaced with the medium supplemented with 100 units/Mℓ of penicillin-streptomycin, followed by further culture for 48 hours. Then, the culture medium was obtained.

### <2-2> Preparation of fibulin-3 over-expressing medium for purification

The present inventors prepared the culture medium in which the cells transfected with the fibulin-3 protein vector for purification (his-fibulin-3) of Example <1-2> were cultured.

Particularly, 5×10⁵ CHO-K1 cells (Korea Research Institute of Bioscience and Biotechnology, Daejeon, Korea) or 293T cells (Hanyang University, Seoul, Korea), which were both easy for protein purification, were distributed in 60 mm plate, followed by transfection with 4 µg of his-fibulin-3 vector using lipofectamine 2000 in penicillin-streptomycin solution (Hyclone) free DMEM:F12 medium (Gibco) and DMEM(Hyclone). After 12 hours of the reaction, the medium was replaced with the medium supplemented with 100 units/Mℓ of penicillin-streptomycin, followed by further culture for 48 hours. Then, each of the culture medium was obtained.

### <2-3> Purification of his-fibulin-3 protein

The present inventors purified his-fibulin-3 protein from the fibulin-3 protein purification medium prepared in Example <2-2>.

Particularly, to purify his-fibulin-3 protein, his-fibulin-3 vector was over-expressed in CHO-K1 cells and 293T cells, which was loaded in protein lysis buffer, followed by pipetting. Reaction was induced at 4°C for 15 ∼ 20 minutes, followed by centrifugation at 4°C at 13000 rpm to separate pellet and supernatant. His beads (Qiagen) washed with PBS twice were added to the supernatant to induce conjugation of his-filuin-3 protein with the beads for 2 hours at 4°C. Then, the beads were collected by centrifugation (4°C, 3000 rpm) and washed with PBS three times. After completely eliminating PBS solution from the beads, elution buffer (250 mM imidazol) was added to the beads, followed by reaction at room temperature for 5 minutes. His-fibulin-3 protein was purified by using centrifugation and the purified protein was transferred new tubes for further use. Protein amount was measured at 595 nm using protein quantification kit (Bio-rad) by Bradford method and the protein was stored at 4°C.

### Example 3: Separation of ALDH1 active cells and ALDH1 inactive cells from A549 cells

The present inventors distinguished and selected cancer stem cells from the non-small-cell lung cancer cell line A549 based on aldehyde dehydrogenase (ALDH) activity by using ALDEFLUOR (Stem Cell Co.).

Particularly, dried ALDEFLUOR reagent was added in 25 µℓ of DMSO (Dimethylsulphoxide), followed by reaction at room temperature for 1 minute until the reagent turned from red-orange to light yellow, indicating that reagent was activated. 25 µℓ of 2 M HCl (hydrochloric acid) was added to the activated reagent, followed by reaction at room temperature for 15 minutes. Then, 360 µℓ of ALDEFLUOR assay buffer was added thereto, which was stored in a refrigerator to prepare ALDEFLUOR substrate. Centrifugation was performed with 1×10⁶ A549 cells and as a result, supernatant was separated. The separated supernatant was discarded and 1 ml of ALDEFLUOR assay buffer was added thereto. Two empty tubes were prepared, to which 500 µℓ of the sample comprising the said cells and ALDEFLUOR assay buffer was added. One of the tubes was added with 5 µℓ of the activated ALDEFLUOR substrate and the other tube was added with the control that was 5 µℓ of DEAB (diethylaminobenzaldehyde) solution. The two tubes were reacted at 37°C, followed by centrifugation to eliminate supernatant. 500 µℓ of ALDEFLUOR assay buffer was added to the cells, followed by reaction at 4°C for 24 hours. Negative charged ALDH-substrate BAAA is infiltrated into live cells via passive diffusion, which is then converted into BAA- by intracellular ALDH with emitting fluorescence. Therefore, the cells emitting fluorescence, measured by FACS sorter (BD bioscience, United states), were distinguished as active cells, while the cells not-emitting fluorescence were distinguished as inactive cells (Figure 2).

### Experimental Example 1: Over-expression of fibulin-3 protein by his-fibulin-3 vector

To confirm whether or not fibulin-3 protein was over-expressed by his-fibulin-3 vector for protein purification (his-fibulin-3), the present inventors first induced over-expression of the vector, followed by PCR and Western blotting.

Particularly, 5×10⁵ CHO-K1 cells (Korea Research Institute of Bioscience and Biotechnology, Daejeon, Korea) or 293T cells (Hanyang University, Seoul, Korea), which were both easy for protein purification, were distributed in 60 mm plate, followed by transfection with 4 µg of his-fibulin-3 vector using lipofectamine 2000 in penicillin-streptomycin solution (Hyclone) free medium. 12 hours later, the medium was replaced with the medium supplemented with 100 units/Mℓ of penicillin-streptomycin, followed by further culture for 48 hours. Fibulin gene expression in CHO-K1 cells transfected with his-fibulin-3 vector was confirmed. First, to extract RNA, cells were mixed with 1 Mℓ of trizol for 5 minutes. Then, 200 µℓ of chloroform reagent was added thereto, followed by mixing for 5 minutes. The mixture was centrifuged at 4°C for 10 minutes. As a result, 200 µℓ of supernatant was obtained. The supernatant was transferred into a new tube. 500 µℓ of isopropanol was added to the obtained supernatant, followed by reaction at room temperature for 10 minutes. Centrifugation was performed at 4°C to discard the supernatant and the precipitate was washed with DEPC containing 75% ethanol. Centrifugation was performed again and the supernatant was discarded. The precipitate obtained from the centrifugation was dissolved in DEPC solution, from which RNA was obtained. After quantification of the RNA, cDNA was synthesized from 1 µg of the RNA by using PCR machine (Takara, Japan) with oligo dT cDNA kit (Intron) (45°C for 1 hr, and 95°C for 5 min). Then, PCR was performed with the primers listed in Table 1 and I-taq polymerase (Intron) as follows; predenaturation at 94°C for 5 minutes, denaturation at 94°C for 30 seconds, annealing at 56°C for 30 seconds, extension at 72°C for 30 seconds, 30 cycles from denaturation to extension, and final extension at 72°C for 10 minutes. The PCR product was loaded onto 1% agarose-gel for electrophoresis.

To confirm the expression of his-fibulin-3 protein, CHO-K1 cells transfected with the vector were collected, to which 50 µℓ of lysis buffer was added. Reaction was induced at 4°C for 30 minutes, followed by centrifugation at 4°C. As a result, supernatant was obtained. 40 µg of each protein quantified by using protein quantification kit (Sigma) was loaded on SDS-gel. The protein loaded on SDS-gel was transferred onto nitrocellulose membrane, followed by reaction at room temperature in BSA buffer for 30 minutes to inhibit non-specific antibody binding. The membrane was reacted with primary antibody anti-β-catenin antibody (Santa Cruz) and anti-β-actin antibody (Sigma) diluted in PBS buffer (1:1000) for 4 hours. The membrane was then reacted with secondary antibody anti-mouse (Cell Signaling) diluted in PBS buffer (1:10000) for 1 hour. The nitrocellulose membrane was washed with PBS 5 times, followed by exposing on film using detection solution.

As a result, it was confirmed that fibulin-3 protein was over-expressed in CHO-K1 cells introduced with his-fibulin-3 vector (Figure 3).

### Experimental Example 2: Characteristics of ALDH1 active cells and ALDH1 inactive cells

### <2-1> Growth of ALDH1 active cells and ALDH1 inactive cells

To compare the cell growth rate between A549 cells and ALDH1 active cells or ALDH1 inactive cells isolated therefrom, the present inventors performed colony formation test with ALDH1 active and inactive cells.

Particularly, A549 cells, ALDH1 active cells or ALDH1 inactive cells isolated therefrom were distributed in 35 mm plate at the density of 1×10³ cells/ml, followed by culture in a 37°C CO₂ incubator for 8 days. Those cells having formed a colony were stained with 0.5% crystal violet reagent for 10 minutes, and then washed with PBS several times, followed by observation under microscope.

As a result, ALDH1 active cells isolated from A549 cells demonstrated better cell growth than A549 cells, while ALDH1 inactive cells were hardly grown (Figure 4).

### <2-2> Expressions of fibulin-3 and β-catenin in ALDH1 active cells and ALDH1 inactive cells

The present inventors also investigated the expressions of fibulin-3 and β-catenin in ALDH1 active cells and ALDH1 inactive cells isolated from the control A549 cells.

Particularly, to extract RNA from A549 cells and ALDH1 active cells and ALDH1 inactive cells isolated therefrom, 1 Mℓ of trizol was added to each cell line, followed by well-mixing for 5 minutes. Then, 200 µℓ of chloroform reagent was added thereto, followed by mixing for 5 minutes. The mixture was centrifuged at 4°C for 10 minutes. As a result, 200 µ*ℓ* of supernatant was obtained. The supernatant was transferred into a new tube. 500 µℓ of isopropanol was added to the obtained supernatant, followed by reaction at room temperature for 10 minutes. Centrifugation was performed at 4°C to discard the supernatant and the precipitate was washed with DEPC containing 75% ethanol. Centrifugation was performed again and the supernatant was discarded. The precipitate obtained from the centrifugation was dissolved in DEPC solution, from which RNA was obtained. After quantification of the RNA, cDNA was synthesized from 1 µg of the RNA by using PCR machine (Takara, Japan) with oligo dT cDNA kit (Intron) (45°C for 1 hr, and 95°C for 5 min). Then, PCR was performed with the primers listed in Table 1 and I-taq polymerase (Intron) as fellow; predenaturation at 94°C for 5 minutes, denaturation at 94°C for 30 seconds, annealing at 56°C for 30 seconds, extension at 72°C for 30 seconds, 30 cycles from denaturation to extension, and final extension at 72°C for 10 minutes. The PCR product was loaded onto 1% agarose-gel for electrophoresis.

**[Table 1]**

| Primer | Sequence(5'-3') | SEQ. ID. NO: |
|---|---|---|
| ALDH forward primer | ATGTCATCCTCAGGCACGCC | 4 |
| ALDH reverse primer | | 5 |
| | | |
| Fibulin-3 forward primer | ATGTTGAAAGCCCTTTTCC | 6 |
| Fibulin-3 reverse primer | CTAAAATGAAAATGGCCCC | 7 |
| β-catenin forward primer (Mod Pathol 2002 ; 15 (4) : 454 - 461) | | 8 |
| β-catenin reverse primer (Mod Pathol 2002 ; 15 (4) : 454 - 461*)* | | 9 |
| β-actin forward primer | | 10 |
| β-actin reverse primer | | 11 |

As a result, it was confirmed that the expression levels of ALDH1 and β-catenin were higher in ALDH1 active cells than those in ALDH1 inactive cells. On the other hand, fibulin-3 expression was greater in inactive cells than that in active cells (Figure 5).

### Experimental Example 3: Inhibitory effect of fibulin-3 on β-catenin expression

### <3-1> β-catenin expression in non-small-cell lung cancer cells

The present inventors investigated the expressions of β-catenin nucleic acid and protein in non-small-cell lung cancer cells A549 and H460.

Particularly, to extract RNA from A549 cells and H460 cells, 1 Mℓ of trizol was added to each cell line, followed by well-mixing for 5 minutes. Then, 200 µℓ of chloroform reagent was added thereto, followed by mixing for 5 minutes. The mixture was centrifuged at 4°C for 10 minutes. As a result, 200 µℓ of supernatant was obtained. The supernatant was transferred into a new tube. 500 µℓ of isopropanol was added to the obtained supernatant, followed by reaction at room temperature for 10 minutes. Centrifugation was performed at 4°C to discard the supernatant and the precipitate was washed with DEPC containing 75% ethanol. Centrifugation was performed again and the supernatant was discarded. The precipitate obtained from the centrifugation was dissolved in DEPC solution, from which RNA was obtained. After quantification of the RNA, cDNA was synthesized from 1 µg of the RNA by performing reverse transcription polymerase chain reaction (RT-PCR) with cDNA Synthesis Kit (Intron Biotechnology, Gyungki-do, Korea). Then, PCR was performed with the β-catenin primers listed in Table 1 and I-taq polymerase (Intron) as follows; predenaturation at 94°C for 5 minutes, denaturation at 94°C for 30 seconds, annealing at 56°C for 30 seconds, extension at 72°C for 30 seconds, 30 cycles from denaturation to extension, and final extension at 72°C for 10 minutes. The PCR product was loaded onto 1% agarose-gel for electrophoresis.

Western blotting was performed as follow. 50 µℓ of lysis buffer was added to each cell line, followed by reaction at 4°C for 30 minutes. Then, centrifugation was performed at 4°C to obtain supernatant. 40 µg of each protein quantified by using protein quantification kit (Sigma) was loaded on SDS-gel. The protein loaded on SDS-gel was transferred onto nitrocellulose membrane, followed by reaction at room temperature in BSA buffer for 30 minutes to inhibit non-specific antibody binding. The membrane was reacted with primary antibody anti-β-catenin antibody (Santa Cruz) and anti-β-action antibody (Sigma) diluted in PBS buffer (1:1000) for 4 hours. The membrane was then reacted with secondary antibody anti-mouse (Cell Signaling) diluted in PBS buffer (1:10000) for 1 hour. The nitrocellulose membrane was washed with PBS 5 times, followed by exposing on film using detection solution.

As a result, it was confirmed that the expressions of β-catenin nucleic acid and protein were higher in A549 cells than in H460 cells (Figure 6).

### <3-2> Inhibitory effect of fibulin-3 on β-catenin expression in non-small-cell lung cancer cells

The present inventors further investigated whether or not fibulin-3 could affect β-catenin nucleic acid and protein expressions in non-small-cell lung cancer cells.

Particularly, A549 cells displaying high expression of β-catenin were transfected with the fibluin-3 gene over-expressing clone constructed in Example <1-1>. In order to suppress fibulin-3 expression in H460 cells demonstrating comparatively low β-catenin, expression, 25 mer of the forward primer UAGAAUGUAGGGAUCUUGACAAGG-3' (SEQ. ID. NO: 12) and the reverse primer 5'-CCrJUGUCAAGAUCCCUACAUUCUAA-3' (SEQ. ID. NO: 13) for fibluin-3 gene, which was fibulin-3 stealth-RNAi, was obtained from Invitrogen Co. 2×10⁵ H460 cells were transfected with 100 nM of the siRNA by using lipofectamine RNAi MAX (Invitrogen). For the negative control (siControl), the cells were transfected with 100 nM of Scrambled Stealth™ RNA in penicillin-streptomycin free medium by using Lipofectamine RNAi MAX (Invitrogen). 4 ∼ 6 hours after the transfection, the medium was replaced with the fresh medium supplemented with 100 units/Mℓ of penicillin-streptomycin, followed by culture for 72 hours. With the cells, PCR using fibulin-3 primer and Western blotting using fibulin-3 antibody (ab14926, Abcam, England) were performed according to the method described in Experimental Example <2-1>. PCR using β-catenin primer and Western blotting using β-catenin antibody were also performed.

As a result, fibulin-3 was over-expressed in A549 cells by fibulin-3 gene over-expressing clone, while fibulin-3 expression was suppressed in H460 cells by fibulin-3 siRNA. β-catenin expression was reduced in A549 cells over-expressing fibulin-3, while β-catenin expression was increased in H460 cells displaying suppressed fibulin-3 expression (Figure 7).

Therefore, it was confirmed that β-catenin expression in non-small-cell lung cancer cell line was inhibited by fibulin-3.

### Experimental Example 4: Effect of fibulin-3 protein on cancer cells

### <4-1> Inhibitory effect of fibulin-3 on β-catenin expression in non-small-cell lung cancer cells

The present inventors investigated the inhibitory effect of fibulin-3 on β-catenin in the non-small-cell lung cancer cell line A549.

Particularly, a mixed medium was prepared by mixing the fibulin-3 over-expressing medium prepared in Example <2-1> and A549 cell culture medium at the ratio of 1 : 4. Another mixed medium was also prepared by mixing H460 culture medium and A549 culture medium at the ratio of 1 : 4. A549 cells (2×10⁵ cells/Mℓ) were treated with those media, followed by observation under microscope. Then, the expression of β-catenin was confirmed by Western blotting by the same manner as described in Experimental Example <3-1>.

As a result, it was confirmed that the expression of β-catenin was suppressed in the cells treated with the mixed medium comprising H460 culture medium and A549 culture medium at the ratio of 1:4 or the mixed medium comprising fibulin-3 over-expressing medium and A549 culture medium at the ratio of 1 : 4, compared that in the A549 cells non-treated (Figure 8).

### <4-2> Inhibitory effect of fibulin-3 on MMP-2 and MMP-7 expressions in non-small-cell lung cancer cells

The present inventors confirmed the inhibitory effect of fibulin-3 on the expressions of MMP-2 and MMP-7 in the lung cancer cell line A549.

Particularly, a mixed medium was prepared by mixing the fibulin-3 over-expressing medium prepared in Example <2-1> and A549 culture medium at the ratio of 1:4. Another mixed medium was also prepared by mixing H460 culture medium in which 5×10⁵ H460 cells showing higher fibulin-3 expression than A549 cells were cultured for 48 hours and A549 culture medium at the ratio of 1:4. Then, A549 cells (2×10⁵ cells/Mℓ) were treated with those media. 48 hours later, Western blotting was performed using anti-MMP-2 antibody (1:1000, cell signaling) and anti-MMP-7 antibody (1:1000, R&D system) to confirm protein expression. To confirm whether or not fibulin-3 could direct affect the expression, the said media were treated with fibulin-3 antibody (500:1) to inhibit the activity of fibulin-3. A549 cells were treated with the media in which fibulin-3 activity was suppressed. 48 hours later, Western blotting was performed using anti-MMP-2 antibody, anti-MMP-7 antibody, and anti-β-catenin antibody to confirm protein expression.

As a result, like β-catenin protein expression, the expressions of MMP-2 and MMP-7 were reduced in the cells treated with the mixed medium comprising H460 culture medium and H549 culture medium at the ratio of 1:4 or the mixed medium comprising fibulin-3 over-expressing medium and A549 culture medium at the ratio of 1:4, compared with those in the control A549 cells. However, the expressions of MMP-2, MMP-7, and β-catenin in the A549 cells treated with the medium which was treated with fibulin-3 antibody (500:1) were similar to those in the control, confirmed by Western blotting, suggesting that the suppression of MMP-2, 7, and β-catenin expression was attributed to fibulin-3 protein (Figure 9).

### <4-3> Inhibitory effect of fibulin-3 on non-small-cell lung cancer cell growth

The present inventors also investigated the inhibitory effect of fibulin-3 on the growth of the non-small-cell lung cancer cell line A549.

Particularly, a mixed medium was prepared by mixing the fibulin-3 over-expressing medium prepared in Example <2-1> and A549 culture medium at the ratio of 1:4. Another mixed medium was also prepared by mixing H460 culture medium in which 5×10⁵ H460 cells showing higher fibulin-3 expression than A549 cells were cultured for 48 hours and A549 culture medium at the ratio of 1:4. Then, A549 cells were treated with those media. 1×10³ A549 cells were distributed on 35 mm plate. 24 hours later, the cells were treated with the mixed medium prepared by mixing fibulin-3 3 over-expressing medium and A549 culture medium at the ratio of 1:1 (50%), the mixed medium prepared by mixing fibulin-3 over-expressing medium and A549 culture medium at the ratio of 5:4 (80%), or fibulin-3 over-expressing medium (100%). The cells were also treated with the mixed medium prepared by mixing H460 culture medium in which 5×10⁵ H460 cells were cultured for 48 hours and A549 culture medium at the ratio of 1:1 (50%), the mixed medium prepared by mixing H460 culture medium and A549 culture medium at the ratio of 5:4 (80%), or H460 culture medium (100%). Then, the cells were cultured in a 37°C CO₂ incubator for 8 days. The cells were stained with 0.5% crystal violet for 10 minutes and washed with PBS several times.

As a result, it was confirmed that the growth of the A549 cells treated with the mixed medium comprising higher ratio of H460 culture medium in which 5×10⁵ H460 cells were cultured for 48 hours or fibulin-3 over-expressing medium was inhibited, compared with that of the control A549 cells (Figure 10) .

In addition, the present inventors confirmed the cell growth inhibitory effect of fibulin-3 via colony formation assay which was performed by treating A549 cells with the his-fibulin-3 over-expressing medium obtained by transfection with the his-fibulin-3 vector for protein purification constructed in Example <2-2>.

Particularly, the his-fibulin-3 over-expressing medium prepared in Example <2-2> using CHO-K1 cells and the his-fibulin-3 over-expressing medium prepared in Example <2-2> using 293T cells were mixed at the ratio of 1:1, to which 1×10³ A549 cells were treated, followed by culture in a 37°C CO₂ incubator for 7 days. The cells were stained with 0.5% crystal violet for 10 minutes and then washed with PBS several times. As a result, it was confirmed that the growth of the A549 cells treated with his-fibulin-3 over-expressing medium was suppressed, compared with that of the control cells (Figure 11).

### <4-4> Inhibitory effect of purified fibulin-3 protein on cancer cell growth

The present inventors also confirmed the inhibitory effect of the purified fibulin-3 protein on cancer cell growth.

Particularly, 1×10³ A549 cells (non-small-cell lung cancer cells) and MDA-MB231 cells (breast cancer cells) were inoculated on 35 mm plate. 24 hours later, the his-fibulin-3 purified protein of Example <2-3> was treated to the lung cancer cells and breast cancer cells at the concentration of 1 µg respectively once or twice (In that case, second treatment was performed 24 hours later on the same spot). Then, the cells were cultured in a 37°C incubator for 7 days. Upon completion of the culture, the cells were stained with 0.5% crystal violet for 10 minutes and then washed with PBS several times.

As a result, it was confirmed that the cell growth was inhibited in both the lung cancer cell line A549 and the breast cancer cell line MDA-MB231 (Figure 12 and Figure 13).

### Experimental Example 5: Effect of fibulin-3 on cancer stem cells

### <5-1> Inhibitory effect of fibulin-3 on MMP-2, MMP-7, and β-catenin expressions in cancer stem cells

The present inventors investigated the inhibitory effect of fibulin-3 on the expressions of MMP-2, MMP-7, and β-catenin in the cancer stem cell line active ALDH1.

Particularly, a mixed medium was prepared by mixing the fibulin-3 over-expressing medium prepared in Example <2-1> and ALDH1 active cell culture medium at the ratio of 1:4. Another mixed medium was also prepared by mixing H460 culture medium in which 5×10⁵ H460 cells showing higher fibulin-3 expression than A549 cells were cultured for 48 hours and ALDH1 active cell culture medium at the ratio of 1:4. Then, ALDH1 active cells (2×10⁵ cells/Mℓ) were treated with those media. 48 hours later, Western blotting was performed by the same manner as described in Example <2-1> using anti-MMP-2 antibody (Cell Signaling), anti-MMP-7 antibody (R&D System), and anti-β-catenin antibody (Santa Cruz) to confirm protein expression. To confirm whether or not fibulin-3 could direct affect the expression, the said media were treated with fibulin-3 antibody (500:1) to inhibit the activity of fibulin-3. ALDH1 active cells were treated with the media in which fibulin-3 activity was suppressed. 48 hours later, Western blotting was performed using anti-MMP-2 antibody, anti-MMP-7 antibody, and anti-β-catenin antibody to confirm protein expression.

As a result, like β-catenin protein expression, the expressions of MMP-2 and MMP-7 were reduced in the cells treated with the mixed medium comprising H460 culture medium and ALDH1 active cell culture medium at the ratio of 1:4 or the mixed medium comprising fibulin-3 over-expressing medium and ALDH1 active cell culture medium at the ratio of 1:4, compared with those in the control ALDH1 active cells. However, the expressions of MMP-2, MMP-7, and β-catenin in the cells treated with the medium which was treated with fibulin-3 antibody (500:1) were similar to those in the control, confirmed by Western blotting, suggesting that the suppression of MMP-2, 7, and β-catenin expressions was attributed to fibulin-3 protein (Figure 14).

### <5-2> Inhibitory effect of fibulin-3 on caner stem cell growth

The present inventors further investigated the inhibitory effect of fibulin-3 on the growth of the caner stem cell line active ALDH1.

Particularly, a mixed medium was prepared by mixing the fibulin-3 over-expressing medium prepared in Example <2-1> and ALDH1 active cell culture medium at the ratio of 1:4. Another mixed medium was also prepared by mixing H460 culture medium in which 5×10⁵ H460 cells showing higher fibulin-3 expression than A549 cells were cultured for 48 hours and ALDH1 active cell culture medium at the ratio of 1:4. Then, ALDH1 active cells were treated with those media. 1×10³ ALDH1 active cells were distributed on 35 mm plate. 24 hours later, the cells were treated with the mixed medium prepared by mixing fibulin-3 over-expressing medium and ALDH1 active cell culture medium at the ratio of 1:1 (50%), the mixed medium prepared by mixing fibulin-3 over-expressing medium and ALDH1 active cell culture medium at the ratio of 5:4 (80%), or fibulin-3 over-expressing medium (100%). The cells were also treated with the mixed medium prepared by mixing H460 culture medium in which 5×10⁵ H460 cells were cultured for 48 hours and ALDH1 active cell culture medium at the ratio of 1:1 (500), the mixed medium prepared by mixing H460 culture medium and ALDH1 active cell culture medium at the ratio of 5:4 (800), or H460 culture medium in which 5×10⁵ H460 cells were cultured for 48 hours (100%). Then, the cells were cultured in a 37°C CO₂ incubator for 8 days. The cells were stained with 0.5% crystal violet for 10 minutes and washed with PBS several times.

As a result, it was confirmed that the growth of the ALDH1 active cells treated with the mixed medium comprising higher ratio of H460 culture medium or fibulin-3 over-expressing medium was inhibited, compared with that of the control ALDH1 active cells (Figure 15).

In addition, the present inventors investigated the inhibitory effect of the his-fibulin-3 over-expressing medium prepared in Example <2-2> on the growth of the cancer stem cell line active ALDH1.

Particularly, the his-fibulin-3 over-expressing medium prepared in Example <2-2> using CHO-K1 cells and the his-fibulin-3 over-expressing medium prepared in Example <2-2> using 293T cells were mixed at the ratio of 1:1, to which 1×10³ ALDH1 active cells were treated, followed by culture in a 37°C CO₂ incubator for 7 days. The cells were stained with 0.5% crystal violet for 10 minutes and then washed with PBS several times, following by performing colony forming assay.

As a result, it was confirmed that the growth of the ALDH1 active cells treated with his-fibulin-3 over-expressing medium was suppressed, compared with that of the control cells (Figure 16).

### <5-3> Inhibitory effect of purified fibulin-3 on caner stem cell growth

The present inventors also investigated the inhibitory effect of the purified fibulin--3 protein on cancer stem cell growth.

Particularly, 1×10³ ALDH1 active cells (cancer stem cells) were inoculated on 35 mm plate. 24 hours later, the his-fibulin-3 purified protein of Example <2-3> was treated to the cells at the concentration of 1 µg once or twice (In that case, second treatment was performed 24 hours later on the same spot). Then, the cells were cultured in a 37°C incubator for 7 days. Upon completion of the culture, the cells were stained with 0.5% crystal violet for 10 minutes and then washed with PBS several times.

As a result, it was confirmed that the growth of the cancer stem cell line active ALDH1 was inhibited (Figure 17).

### Manufacturing Example 1: Preparation of pharmaceutical formulations

### <1-1> Preparation of powders

| | |
|---|---|
| Fibulin-3 protein | 2 g |
| Lactose | 1 g |

Powders were prepared by mixing all the above components, which were filled in airtight packs according to the conventional method for preparing powders.

### <1-2> preparation of tablets

| | |
|---|---|
| Fibulin-3 protein | 100 mg |
| Corn starch | 100 mg |
| Lactose | 100 mg |
| Magnesium stearate | 2 mg |

Tablets were prepared by mixing all the above components by the conventional method for preparing tablets.

### <1-3> Preparation of capsules

| | |
|---|---|
| Fibulin-3 protein | 100 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

### <1-4> Preparation of pills

| | |
|---|---|
| Fibulin-3 protein | 1 g |
| Lactose | 1.5 g |
| Glycerin | 1 g |
| Xylitol | 0.5 g |

Pills were prepared by mixing all the above components according to the conventional method for preparing pills. Each pill contained 4 g of the mixture.

### <1-5> Preparation of granules

| | |
|---|---|
| Fibulin-3 protein | 150 mg |
| Soybean extract | 50 mg |
| Glucose | 200 mg |
| Starch | 600 mg |

All the above components were mixed, to which 100 mg of 30% ethanol was added. The mixture was dried at 60°C and the prepared granules were filled in packs.

## Claims

1. A pharmaceutical composition comprising fibulin-3 protein for use in preventing the recurrence of a lung or breast cancer, wherein the fibulin-3 protein comprises the amino acid sequence of SEQ. ID. NO: 1.

2. A pharmaceutical composition comprising a fibulin-3 protein over-expressing cell line for use in preventing the recurrence of a lung or breast cancer, wherein the fibulin-3 protein comprises the amino acid sequence of SEQ. ID. NO: 1.

3. The pharmaceutical composition for use according to claim 1 or 2, wherein the fibulin-3 protein inhibits the growth of a lung or breast cancer stem cell.

4. The pharmaceutical composition for use according to claim 3, wherein the lung or breast cancer stem cell is selected by the marker selected from the group consisting of CD133 (prominin-1; AC133), CD44 (hyaluronate receptor; P-glycoprotein 1), and ALDH1 (Aldehyde dehydrogenase 1).

## Patentansprüche

1. Eine pharmazeutische Zusammensetzung, welche Fibulin-3 Protein umfasst, zur Verwendung in der Vermeidung des Wiederauftretens eines Lungen- oder Brustkrebses, wobei das Fibulin-3 Protein die Aminosäuresequenz von SEQ ID NO.: 1 umfasst.

2. Eine pharmazeutische Zusammensetzung, welche eine Fibulin-3 Protein überexprimierende Zelllinie umfasst, zur Verwendung in der Vermeidung des Wiederauftretens eines Lungen- oder Brustkrebses, wobei das Fibulin-3 Protein die Aminosäuresequenz von SEQ ID NO.: 1 umfasst.

3. Die pharmazeutische Zusammensetzung gemäß Anspruch 1 oder 2, wobei das Fibulin-3 Protein das Wachstum einer Lungen- oder Brustkrebsstammzelle inhibiert.

4. Die pharmazeutische Zusammensetzung zur Verwendung gemäß Anspruch 3, wobei die Lungen- oder Brustkrebsstammzelle ausgewählt ist durch den Marker ausgewählt aus der Gruppe bestehend aus CD133 (Prominin-1; AC133), CD44 (Hyaluronatrezeptor; P-Glykoprotein 1), und ALDH1 (Aldehyd-Dehydrogenase 1).

## Revendications

1. Composition pharmaceutique comprenant la protéine fibuline 3 pour l'utilisation dans la prévention de la récurrence d'un cancer du poumon ou du sein, la protéine fibuline 3 comprenant la séquence d'acides aminés de SEQ ID n° : 1.

2. Composition pharmaceutique comprenant une lignée cellulaire surexprimant la protéine fibuline 3 pour l'utilisation dans la prévention de la récurrence d'un cancer du poumon ou du sein, la protéine fibuline 3 comprenant la séquence d'acides aminés de SEQ ID n° : 1.

3. Composition pharmaceutique pour l'utilisation selon la revendication 1 ou 2, dans laquelle la protéine fibuline 3 inhibe le développement d'une cellule souche cancéreuse du poumon ou du sein.

4. Composition pharmaceutique pour l'utilisation selon la revendication 3, dans laquelle la cellule souche cancéreuse du poumon ou du sein est sélectionnée par le marqueur sélectionné dans le groupe constitué de CD133 (prominine 1 ; AC133), CD44 (récepteur hyaluronate ; glycoprotéine P 1), et ALDH1 (aldéhyde déshydrogénase 1).


### SEQUENCE LISTING

<110> Korea Atomic Energy Research Institute
<120> PHARMACEUTICAL COMPOSITION CONTAINING FIBULIN-3 PROTEIN AS AN ACTIVE INGREDIENT FOR INHIBITING THE GROWTH OF CANCER STEM CELLS
<130> EP91160HV163pau
<140> EP11870806.4
   <141> 2011-12-22
<150> PCT/KR2011/010001
   <151> 2011-12-22
<150> KR10-2011-0079033
   <151> 2011-08-09
<160> 15
<170> PatentIn version 3.5
<210> 1
   <211> 493
   <212> PRT
   <213> Homo sapiens
<400> 1
<210> 2
   <211> 23
   <212> DNA
   <213> Artificial Sequence
<220>
   <223> fibulin-3 vector forward
<400> 2
   atataagctt atgttgaaag ccc 23
<210> 3
   <211> 29
   <212> DNA
   <213> Artificial Sequence
<220>
   <223> fibulin-3 vector reverse
<400> 3
   atatctcgag ctaaaatgaa aatggcccc 29
<210> 4
   <211> 20
   <212> DNA
   <213> Artificial Sequence
<220>
   <223> ALDH1 RT-PCR forward
<400> 4
   atgtcatcct caggcacgcc 20
<210> 5
   <211> 23
   <212> DNA
   <213> Artificial Sequence
<220>
   <223> ALDH1 RT-PCR reverse
<400> 5
   ttatgactgt gactgttttg acc 23
<210> 6
   <211> 19
   <212> DNA
   <213> Artificial Sequence
<220>
   <223> fibulin-3 RT-PCR forward
<400> 6
   atgttgaaag cccttttcc 19
<210> 7
   <211> 19
   <212> DNA
   <213> Artificial Sequence
<220>
   <223> fibulin-3 RT-PCR reverse
<400> 7
   ctaaaatgaa aatggcccc 19
<210> 8
   <211> 23
   <212> DNA
   <213> Artificial Sequence
<220>
   <223> beta-catenin RT-PCR forward
<400> 8
   gctgatttga tggagttgga cat 23
<210> 9
   <211> 22
   <212> DNA
   <213> Artificial Sequence
<220>
   <223> beta-catenin RT-PCR reverse
<400> 9
   gacttgggag gtatccacat cc 22
<210> 10
   <211> 21
   <212> DNA
   <213> Artificial Sequence
<220>
   <223> beta-actin RT-PCR forward
<400> 10
   catcctcacc ctgaagtacc c 21
<210> 11
   <211> 22
   <212> DNA
   <213> Artificial Sequence
<220>
   <223> beta-actin RT-PCR reverse
<400> 11
   agcctggata gcaacgtaca tg 22
<210> 12
   <211> 24
   <212> DNA
   <213> Artificial Sequence
<220>
   <223> sifibulin-3 forward
<400> 12
   uagaauguag ggaucuugac aagg 24
<210> 13
   <211> 25
   <212> DNA
   <213> Artificial Sequence
<220>
   <223> sifibulin-3 reverse
<400> 13
   ccuugucaag aucccuacau ucuaa 25
<210> 14
   <211> 35
   <212> DNA
   <213> Artificial Sequence
<220>
   <223> his-fibulin-3 forward
<400> 14
   cggaattcat gttgaaagcc cttttcctaa ctatg 35
<210> 15
   <211> 43
   <212> DNA
   <213> Artificial Sequence
<220>
   <223> his-fibulin-3 reverse
<400> 15
   ccggctcgag ctagtggtgg tggtggtggt gaaatgaaaa tgg 43